# EUROPÄISCHE PATENTANMELDUNG

(11) **EP 4 664 478 A1**
(43) Veröffentlichungstag der Anmeldung: **17.12.2025**
(21) Anmeldenummer: 25174976.8
(22) Anmeldetag: 08.05.2025
(51) Int. Cl.: G16H 40/63, G16H 30/40, A61B 6/00, G06F 3/04842, G06F 3/04847, A61B 5/055, A61B 6/03

(54) **BILDGEBUNGSVORRICHTUNG UND VERFAHREN ZUR BESTIMMUNG EINES BILDGEBUNGSPROTOKOLLS FÜR EINEN SUBBEREICH**

(30) Priorität: 11.06.2024 EP 24181396
(71) Anmelder: Siemens Healthineers AG, 91301 Forchheim (DE)
(72) Erfinder: Rupp, Dominik, 90587 Tuchenbach (DE); Van de Stadt-Lagemaat, Miriam, 91056 Erlangen (DE); Mehler, Frank, 91077 Neunkirchen am Brand (DE)
(74) Vertreter: Siemens Healthineers Patent Attorneys

(57) **Zusammenfassung**

Die Erfindung betrifft ein Computer-implementiertes Verfahren zur Bestimmung eines Bildgebungsprotokolls zur Erfassung von Bilddaten eines diagnostisch relevanten Subbereichs einer Körperregion eines Patienten mittels einer Bildgebungsvorrichtung, umfassend: Erfassen (S1) einer Information über die Körperregion, Bereitstellen (S2) einer Eingabeoption zum Erfassen einer Information über einen Subbereich der Körperregion in Abhängigkeit der Information über die Körperregion, Erfassen (S3) des diagnostisch relevanten Subbereichs in Abhängigkeit der Eingabeoption zum Erfassen der Information über den Subbereich der Körperregion, Ermitteln (S4) des Bildgebungsprotokolls zur Erfassung von Bilddaten des diagnostisch relevanten Subbereichs der Körperregion des Patienten in Abhängigkeit des erfassten diagnostisch relevanten Subbereichs, Bereitstellen (S5) des Bildgebungsprotokolls zur Erfassung von Bilddaten des diagnostisch relevanten Subbereichs der Körperregion mittels der Bildgebungsvorrichtung. Die Eingabeoption umfasst eine Mehrzahl von Subbereichen der Körperregion. Die Erfindung betrifft ferner eine Bildgebungsvorrichtung zur Erfassung von Bilddaten eines diagnostisch relevanten Subbereichs einer Körperregion eines Patienten und ein Computerprogrammprodukt, welches direkt in eine Speichereinheit einer Recheneinheit einer erfindungsgemäßen Bildgebungsvorrichtung ladbar ist, mit Programmcode-Mitteln, um ein erfindungsgemäßes Computer-implementiertes Verfahren auszuführen.

## Beschreibung

Die Erfindung betrifft ein Verfahren zur Bestimmung eines Bildgebungsprotokolls für eine Bildgebungsuntersuchung mittels einer Bildgebungsvorrichtung. Zudem betrifft die Erfindung eine medizinische Bildgebungsvorrichtung, die zur Aufnahme von Bilddaten einer Körperregion eines Patienten geeignet ist.

Die Planung eines Bildgebungsprotokolls zur Erfassung von Bilddaten einer diagnostisch relevanten Körperregion eines Patienten mittels einer Bildgebungsvorrichtung kann von patientenspezifischen Voraussetzungen und/oder nutzerspezifischen Eingaben bei einer Patientenregistrierung sowie einer Vorbereitung des Patienten für eine Bildgebungsuntersuchung abhängen. Beispielsweise sind bei der Planung des Bildgebungsprotokolls eine Lateralität der diagnostisch relevanten Körperregion (z. B. das Vorhandensein in einer linken oder rechten Körperhälfte), eine Unterteilung der Körperregion in relevante und irrelevante Subbereiche und/oder eine Orientierung der Körperregion in einem Bezugssystem zu beachten. Insbesondere bei Bildgebungsprotokollen für die Zahn- oder Kieferregion des Patienten kann eine Spezifikation eines geeigneten Bildgebungsbereichs, sowie eine Auswahl von relevanten Bereichen einer Körperregion aufgrund des symmetrischen Aufbaus der Zahnbögen (z. B. linker, rechter, oder frontaler Unterkiefer und/oder Oberkiefer) und der geschwungenen Form der Zahnbögen komplex sein. Daher müssen Nutzer der Bildgebungsvorrichtung aufwändig trainiert werden, bzw. über spezifisches Fachwissen und Erfahrungswerte verfügen, um ein fehlerfreies Erfassen von Bilddaten der diagnostisch relevanten Körperregionen mittels einer Bildgebungsvorrichtung im klinischen Alltag zu gewährleisten.

Kommerziell verfügbare Bildgebungsvorrichtungen verfügen typischerweise über ein Planungsprogramm zur Planung eines Bildgebungsprotokolls für eine Bildgebungsuntersuchung einer Körperregion eines Patienten. Solche Planungsprogramme berücksichtigen jedoch selten die Differenziertheit der Körperregion als solche, wie beispielsweise eine Lateralität und/oder Untergliederung der Körperregion. Daher kann durch ein Planungsprogramm beispielsweise eine Bildgebung für die gesamte Hüftregion eines Patienten vorbereitet werden, obwohl nur die rechte Hüfte des Patienten diagnostisch relevant ist. Für einen Anteil an Untersuchungen ist eine solche Grobauswahl einer diagnostisch relevanten Körperregion ausreichend, abhängig von der Komplexität der Diagnosestellung beziehungsweise dem Untersuchungsverlauf müssen im Regelfall jedoch weitere detailliertere Untersuchungen angeschlossen werden.

In einigen Anwendungsfällen werden durch das Planungsprogramm verschiedene Parameter oder Szenarien für den Nutzer voreingestellt, wobei der Nutzer jedoch insbesondere bei Anpassungsdarf mit dem Planungsprogramm vertraut sein, und ein entsprechendes Training absolviert haben muss. Die Komplexität der Bildgebungsplanungen führt überdies zum Teil zu einer ineffizienten bzw. zeitintensiven Bildprotokollermittlung durch beispielsweise eine Duplikation von Anweisungen, Anleitungen, Workflows, die idealerweise vermieden werden können. In anderen Anwendungsfällen bedarf es einer (nahezu vollständig) manuellen Erstellung eines Bildgebungsprotokolls durch einen Nutzer, insbesondere wenn lediglich ein spezifischer Bereich einer Körperregion, beispielweise die Schambein-Region einer Hüftregion des Patienten, durch eine Bildgebungsuntersuchung aufgenommen werden soll.

Es ist eine Aufgabe der vorliegenden Erfindung, die Effizienz einer Bestimmung eines Bildgebungsprotokolls für eine Erfassung von Bilddaten einer Körperregion eines Patienten mittels einer Bildgebungsvorrichtung zu verbessern. Die Aufgabe wird durch die Merkmale der unabhängigen Ansprüche gelöst. Vorteilhafte Ausgestaltungen und zweckmäßige Weiterbildungen sind in den abhängigen Ansprüchen beschrieben.

Das erfindungsgemäße Computer-implementierte Verfahren zur Bestimmung eines Bildgebungsprotokolls zur Erfassung von Bilddaten eines diagnostisch relevanten Subbereichs einer Körperregion eines Patienten mittels einer Bildgebungsvorrichtung umfasst die folgenden Schritte:
- Erfassen einer Information über die Körperregion,
- Bereitstellen einer Eingabeoption zum Erfassen einer Information über einen Subbereich der Körperregion in Abhängigkeit der Information über die Körperregion, wobei die Eingabeoption eine Mehrzahl von Subbereichen der Körperregion umfasst,
- Erfassen des diagnostisch relevanten Subbereichs in Abhängigkeit der Eingabeoption zum Erfassen der Information über den Subbereich der Körperregion,
- Bestimmen des Bildgebungsprotokolls zur Erfassung von Bilddaten des diagnostisch relevanten Subbereichs der Körperregion des Patienten in Abhängigkeit des erfassten diagnostisch relevanten Subbereichs,
- Bereitstellen des Bildgebungsprotokolls zur Erfassung von Bilddaten des diagnostisch relevanten Subbereichs der Körperregion mittels der Bildgebungsvorrichtung.

Das Erfassen von Bilddaten des diagnostisch relevanten Subbereichs der Körperregion des Patienten mittels einer Bildgebungsvorrichtung kann auch als Bildgebungsuntersuchung bezeichnet werden.

Das Verfahren kann bevorzugt ein Erfassen von Bilddaten des diagnostisch relevanten Subbereichs der Körperregion des Patienten mittels des bereitgestellten Bildgebungsprotokolls umfassen. Insbesondere kann das Verfahren ein Durchführen einer Bildgebungsuntersuchung in Abhängigkeit des Bildgebungsprotokolls umfassen. Mit anderen Worten kann durch das Bildgebungsprotokoll die Bildgebungsuntersuchung an der Bildgebungsvorrichtung gesteuert werden. Die Bildgebungsuntersuchung kann ein Ausführen von einer oder mehreren Bildgebungssequenzen umfassen.

Ein Bildgebungsprotokoll kann insbesondere einen oder mehrere Bildgebungsparameter und/oder Bildgebungsparametergruppen umfassen. Beispiele für Bildgebungsparameter sind eine räumliche Auflösung, ein Kontrast, eine Schichtdicke, eine Abmessung eines Bildgebungsvolumens, eine Relaxationszeit, eine Echozeit und dergleichen. Ein Bildgebungsparameter kann insbesondere eine beliebige bildrelevante Einstellung der Bildgebungsvorrichtung, aber auch einen Parameter eines Workflows der Bildgebungsuntersuchung, umfassen. Weiterhin können auch Parameter, welche einen Bildgebungsbereich der Bildgebungsvorrichtung definieren, als Bildgebungsparameter verstanden werden. Ein Bildgebungsvolumen kann beispielsweise ein Volumen mit einer höchsten Homogenität eines Magnetfelds, insbesondere ein Isozentrum, der Magnetresonanzvorrichtung darstellen.

Die Körperregion kann ein Gelenk, einen Knochen, einen Knochenverbund, ein Körperteil, eine Extremität oder dergleichen umfassen. Beispielsweise kann die Körperregion eine Hüftregion, eine Schulterregion, eine Knieregion, eine Hirnregion, eine Augenregion oder eine beliebige andere anatomische Struktur umfassen. Weiterhin kann die Körperregion eine Gewebestruktur oder ein Organ, wie beispielsweise ein Herz, eine Leber, eine Niere, oder dergleichen, umfassen. Die Körperregion kann auch als Körperbereich, Teilbereich eines Körpers, Körperabschnitt oder Körperteil bezeichnet werden. Vorzugsweise umfasst die Körperregion des Patienten eine Kieferregion oder eine Zahnregion.

Die Körperregion umfasst insbesondere eine Mehrzahl von Subbereichen. Ein Subbereich der Körperregion kann auch als Subregion, Teilabschnitt, Unterabschnitt bezeichnet werden. Ein Subbereich umfasst bevorzugt einen Teilbereich der Körperregion. Ein Subbereich kann insbesondere einen Abschnitt der Körperregion darstellen, welcher sich aufgrund von Gewebeeigenschaften und/oder einer anatomischen Struktur, von weiteren Abschnitten oder Subbereichen der Körperregion unterscheidet oder abgrenzt. Beispielsweise können einzelne Glieder eines Fingers oder eines Handknochens Subbereiche der Körperregion des Patienten darstellen.

Ferner kann eine Ausrichtung, eine Lage und/oder eine Erstreckung eines ersten Subbereichs der Körperregion von einer Ausrichtung und/oder Erstreckung eines zweiten Subbereichs der Körperregion abweichen. Es kann erforderlich oder wünschenswert sein, unterschiedliche Bildgebungsbereiche in Abhängigkeit der Ausrichtung, Lage und/oder Erstreckung des ersten Subbereichs und des zweiten Subbereichs der Körperregion zu definieren. Durch ein Einschränken oder Anpassen der Bildgebungsbereiche an eine Mehrzahl von Subbereichen der Körperregion kann eine Dauer einer Bildgebungsuntersuchung der Subregionen auf vorteilhafte Weise reduziert werden. Ist die Körperregion beispielweise die Kieferregion eines Patienten, kann ein Subbereich der Körperregion beispielsweise ein Abschnitt des Unterkieferknochens (corpus mandibulae) und/oder der Bereich des Kieferwinkels (angulus mandibulae) sein. Ein weiteres Beispiel für Subbereiche einer Körperregion können Zähne und/oder Abschnitte eines Zahnbogens sein. Die Subbereiche der Köperregion können aneinander angrenzen, nebeneinander angeordnet sein und/oder sich gegenseitig überlappen und/oder einschließen.

Als diagnostisch relevanter Subbereich kann ein Subbereich einer Körperregion bezeichnet werden, der für eine Untersuchungsaufgabe, Befundung, Behandlung oder Diagnose besonders relevant ist. Mit anderen Worten bezeichnet der diagnostisch relevante Subbereich einen oder mehrere ausgewählte Subbereiche und/oder den Bereich der Körperregion eines Patienten, für den eine Bildgebungsuntersuchung durchgeführt werden soll. Der diagnostisch relevante Subbereich kann insbesondere einen Bereich von Interesse ("region of interest") darstellen. Es ist vorstellbar, dass der diagnostisch relevante Subbereich bezüglich einer Beschaffenheit und/oder Ausdehnung einem oder mehreren Subbereichen der Körperregion des Patienten entspricht. Der diagnostisch relevante Subbereich kann insbesondere eine Mehrzahl von Subbereichen der Körperregion des Patienten umfassen. Der diagnostisch relevante Subbereich der Körperregion des Patienten ist insbesondere ein durch den Nutzer ausgewählter Subbereich aus einer Mehrzahl aus bereitgestellten Subbereichen einer Körperregion.

Bilddaten können insbesondere Daten darstellen, welche mittels der Bildgebungsvorrichtung von der Körperregion und/oder eines Subbereichs des Patienten erfasst werden. Bilddaten können insbesondere ein Ergebnis einer Bildgebungsuntersuchung mittels der Bildgebungsvorrichtung sein. Bilddaten können insbesondere sowohl Rohdaten als auch Bilder, welche aus den Rohdaten abgeleitet werden, umfassen. Beispielsweise können die Bilddaten von einer Magnetresonanzvorrichtung erfasste, digitalisierte Magnetresonanzsignale umfassen. Die Bilddaten können insbesondere als komplexe Werte in einer k-Raum Matrix gespeichert sein. Vorzugsweise umfassen die Bilddaten Magnetresonanzbilder, welche in Abhängigkeit der digitalisierten Magnetresonanzsignale rekonstruiert wurden.

Eine Bildgebungsvorrichtung kann insbesondere eine Vorrichtung zur Erfassung von Bilddaten eines Untersuchungsobjekts, insbesondere einer Körperregion bzw. eines oder mehrerer Subbereiche einer Körperregion eines Patienten sein. Mit anderen Worten ist mittels der Bildgebungsvorrichtung eine Bildgebungsuntersuchung durchführbar. Alternative Bezeichnungen für eine Bildgebungsvorrichtung können Bildgebungsgerät, medizinisches Bildgebungssystem oder Bilderfassungseinheit sein. Vorzugsweise ist eine Bildgebungsvorrichtung dazu ausgebildet, zweidimensionale und/oder dreidimensionale Bilddaten, insbesondere zeitabhängige dreidimensionale Bilddaten, des Untersuchungsobjekts aufzunehmen. Beispiele für Bildgebungsvorrichtungen sind Magnetresonanzgeräte, Röntgengeräte, Computertomografiegeräte, Einzelphotonen-Emissions-Computertomografen, Positron-Emissions-Tomografen, aber auch Mammografie-Geräte, Ultraschallgeräte und dergleichen. In einer bevorzugten Ausführungsform ist die Bildgebungsvorrichtung eine Magnetresonanzvorrichtung.

Eine Information über die Körperregion des Patienten kann eine beliebige Beschreibung eines Typs, einer Bezeichnung, einer Position und/oder einer Ausdehnung der Körperregion des Patienten umfassen. Die Information über die Körperregion kann ferner eine Selektion und/oder eine Identifikation der Körperregion oder einer anatomischen Struktur aus einer Liste oder einer Datenbank von Körperregionen umfassen.

Die Information über die Körperregion des Patienten kann manuell von einem Nutzer der Bildgebungsvorrichtung mittels einer Benutzerschnittstelle eingegeben werden. Insbesondere kann die Information über die Körperregion mittels einer Auswahl einer anatomischen Struktur oder eines Abschnitts einer anatomischen Struktur in Abhängigkeit einer mittels der Benutzerschnittstelle bereitgestellten Repräsentation der anatomischen Struktur durch den Nutzer der Bildgebungsvorrichtung ausgewählt werden. Es ist weiterhin vorstellbar, dass die Bildgebungsvorrichtung eine Steuereinheit und/oder eine Recheneinheit aufweist, die dazu ausgebildet sind, die Information über die Körperregion des Patienten in Abhängigkeit einer Patienteninformation bzw. Patientendaten mittels einer geeigneten Schnittstelle von einem Datennetzwerk, einem medizinischen Informationssystem, einer Cloud und/oder einer lokalen Speichereinheit zu beziehen. Insbesondere kann die Information über die Körperregion mittels eines Algorithmus oder eines Bildverarbeitungsalgorithmus in Abhängigkeit der Patientendaten, Bilddaten einer vorangegangenen Bildgebungsuntersuchung der Körperregion des Patienten und/oder einer Untersuchungsanweisung ermittelt werden.

Es ist vorstellbar, dass die Information über die Körperregion vorbestimmt ist. In diesem Fall kann das Erfassen der Information über die Köperregion automatisiert erfolgen oder ein einmaliges Festlegen der Information über die Körperregion umfassen. Beispielweise kann eine Bildgebungsvorrichtung lediglich für eine Aufnahme und/oder Erfassung von Bilddaten von Kieferregionen von Patienten ausgebildet sein bzw. eingesetzt werden. Insbesondere kann die Bildgebungsvorrichtung als ein dedizierter Scanner, beispielsweise ein Extremitäten-Scanner oder ein Dentalscanner, ausgestaltet sein. In einer Ausführungsform wird die Information über die Körperregion initial (beispielweise in einem Einrichtungsschritt der Bildgebungsvorrichtung in einer medizinischen Einrichtung) erfasst und anschließend für eine oder mehrere folgende Durchführungen des erfindungsgemäßen Verfahrens bzw. Bestimmen des Bildgebungsprotokolls zur Erfassung von Bilddaten des diagnostisch relevanten Subbereichs der Körperregion des Patienten in Abhängigkeit des erfassten diagnostisch relevanten Subbereichs gespeichert.

Das Bereitstellen der Eingabeoption zum Erfassen einer Information über einen Subbereich der Körperregion umfasst vorzugsweise ein Ausgeben der Eingabeoption mittels einer Ausgabeeinheit und/oder einer Benutzerschnittstelle der Bildgebungsvorrichtung. Das Erfassen der Information über die Körperregion kann auch von dem Schritt des Bereitstellens der Eingabeoption zum Erfassen einer Information über einen Subbereich der Körperregion umfasst sein. Beispielweise kann ein Nutzer zunächst eine Körperregion mittels einer grafischen Repräsentation der Körperregionen des Patienten auswählen und unmittelbar nachfolgend die grafische Repräsentation der Körperregion bereitgestellt werden.

Bevorzugt umfasst die Eingabeoption zum Erfassen der Information über den Subbereich der Körperregion eine grafische Repräsentation der Körperregion und/oder einer oder mehrerer Subbereiche der Körperregion des Patienten. Eine Eingabeoption kann einem Nutzer ein Anwählen von ausgegebenen Optionen, insbesondere von Subbereichen der Körperregion, ermöglichen. Insbesondere kann die Eingabeoption zur Selektierung eines oder mehrerer Subbereiche der Körperregion des Patienten ausgebildet sein. Bevorzugt umfasst die Eingabeoption eine Ausgabe von Informationen über einen Subbereich der Körperregion.

Der diagnostisch relevante Subbereich der Körperregion des Patienten kann insbesondere ein durch den Nutzer ausgewählter Subbereich aus einer Mehrzahl von bereitgestellten Subbereichen einer Körperregion, welche mittels der Eingabeoption zum Erfassen der Information über den Subbereich der Körperregion bereitgestellt werden, darstellen.

Beispielsweise kann die Eingabeoption zum Erfassen der Information über den Subbereich der Körperregion zur Auswahl eines oder mehrerer Subbereiche und/oder Anpassung eines Parameters eines Bildgebungsprotokolls des diagnostisch relevanten Subbereichs mittels einer grafischen Benutzerschnittstelle, insbesondere eines Monitors oder eines Touchscreens, an den Nutzer ausgegeben werden. Die Eingabeoption kann beispielweise eine oder mehrere Eingabemasken umfassen. Eine Eingabemaske kann es dem Nutzer ermöglichen, einen Subbereich auszuwählen, einen oder mehrere Bildgebungsbereiche zu bestimmen, einen oder mehrere Parameter eines diagnostisch relevanten Subbereichs und/oder eines oder mehrerer Bildgebungsbereiche anzupassen bzw. zu ändern.

Ein Bildgebungsbereich, auch gemeinsamer Bildgebungsbereich, kann einen oder mehrere diagnostisch relevante Subbereiche der Körperregion des Patienten umfassen. Die Eingabeoption zum Erfassen der Information über den Subbereich der Körperregion kann bevorzugt, insbesondere bei einer Auswahl einer Mehrzahl von Subbereichen, eine grafische Repräsentation des gemeinsamen Bildgebungsbereichs umfassen, um dem Nutzer den gemeinsamen Bildgebungsbereich der diagnostisch relevanten Subbereiche der Körperregion zu verdeutlichen. Aufgrund dieser Information kann der Nutzer bevorzugt dazu veranlasst werden eine ideale Auswahl von diagnostisch relevanten Subbereichen vorzunehmen.

In einem Beispiel umfasst die Information über die Körperregion des Patienten eine Bezeichnung oder eine Identifikation eines Abschnitts einer anatomischen Struktur, insbesondere eines Abschnitts einer Zahn- oder Kieferregion eines Patienten, beispielweise eines Abschnitts eines Oberkiefers (Maxilla). Die Eingabeoption zum Erfassen der Information des Subbereichs kann entsprechend in Abhängigkeit der Information über eine Körperregion mittels einer Ausgabeeinheit oder einer grafischen Benutzerschnittstelle dem Nutzer der Bildgebungsvorrichtung bereitgestellt werden. Beispielsweise kann die Eingabeoption zum Erfassen der Information über den Subbereich aus einer Datenbank einer Speichereinheit, welche eine Mehrzahl von Eingabeoptionen zur Auswahl von Subbereichen für unterschiedliche Körperregionen umfasst, ausgewählt und abgerufen werden. Insbesondere kann eine Recheneinheit und/oder ein Algorithmus in Abhängigkeit der Information über eine Körperregion die Eingabeoption in Form einer Auflistung von Subbereichen und/oder einer grafischen Repräsentation ermitteln und/oder aus einer Datenbank auswählen. Beispielweise kann die Eingabeoption für eine Oberkiefer-Region eines Patienten eine Auswahl von (Knochen-) Subbereichen, wie dem Stirnfortsatz (Processus frontalis), Alveolarfortsatz (Processus alveolaris) oder dem Oberkieferhöcker (Tuber maxillae) umfassen.

Ein Nutzer der Bildgebungsvorrichtung kann beispielsweise ein Mediziner, insbesondere ein Zahnmediziner, ein medizinisch-technischer Assistent oder ein Mitglied eines medizinischen Personals einer Praxis oder einer klinischen Einrichtung sein. Der Nutzer kann an einem Standort der Bildgebungsvorrichtung, aber auch an einem beliebigen anderen Ort positioniert oder befindlich sein. Beispielsweise kann der Nutzer in einer anderen Stadt, einer anderen Region und/oder einem anderen Land sein und mit der Bildgebungsvorrichtung interagieren bzw. diese fernsteuern.

Das Erfassen des diagnostisch relevanten Subbereichs der Körperregion kann ein Erfassen einer Eingabe des Nutzers mittels der Eingabeoption zum Erfassen der Information über den Subbereich der Körperregion umfassen. Das Erfassen des diagnostisch relevanten Subbereichs umfasst insbesondere die Auswahl und/oder Kennzeichnung eines oder mehrerer Subbereiche als diagnostisch relevante Subbereiche durch eine Eingabe des Nutzers mittels der Eingabeoption. Mit anderen Worten kann das Erfassen des diagnostisch relevanten Subbereichs ein Erfassen einer Eingabe des Nutzers als Antwort des Nutzers auf die Eingabeoption darstellen. Die Eingabe des Nutzers kann somit die Information über den Subbereich der Körperregion umfassen. Insbesondere kann die Eingabe des Nutzers eine oder mehrere Subbereiche der Körperregion oder eine Bezeichnung mindestens eines Subbereichs der Körperregion umfassen.

Vorzugsweise wird die Eingabeoption zum Erfassen der Information über den Subbereich der Körperregion mittels einer Eingabeschnittstelle oder einer Benutzerschnittstelle der Bildgebungsvorrichtung bereitgestellt. Beispielweise kann die Eingabeschnittstelle oder die Benutzerschnittstelle eine Maus, eine Tastatur, einen Touchscreen und/oder eine Sprachschnittstelle umfassen. Die Eingabe des Nutzers kann bevorzugt ein Auswählen eines grafischen Objekts, welches einen Subbereich der Körperregion des Patienten repräsentiert, umfassen. Die Eingabe des Nutzers kann bevorzugt ein Auswählen eines textuellen Objekts, beispielsweise ein Auswählen eines Elements aus einer Auslistung, umfassen. Die Eingabe des Nutzers kann durch eine textbasierte Eingabe, beispielsweise durch die Eingabe einer Abkürzung, eines Akronyms oder eines Begriffs für den in ein Eingabefenster, oder eine grafische Eingabe, beispielweise einer grafischen Auswahl mittels eines Cursors in einem grafischen Eingabefenster, erfolgen. Die textbasierte Eingabe kann eine oder mehrere Koordinaten, eine oder mehrere Abmessungen des Subbereichs, eine Koordinate eines geometrischen Mittelpunkts des Subbereichs oder dergleichen umfassen. Die mittels einer Eingabeschnittstelle oder eine Benutzerschnittstelle der Bildgebungsvorrichtung bereitstellte Eingabeoption kann dem Nutzer Informationen bereitstellen, die den Nutzer zu einem Auswählen eines grafischen Objekts, einem Auswählen eines textuellen Objekts, einer textbasierten Eingabe und/oder grafischer Eingabe führen können.

Bevorzugt entspricht der diagnostisch relevante Subbereich den in Abhängigkeit der Eingabe des Nutzers erfassten Subbereichen. Beispielsweise kann der diagnostisch relevante Subbereich mit einer Auswahl von einem oder mehreren Subbereichen durch den Nutzer übereinstimmen. Weiterhin kann der diagnostisch relevante Subbereich auch Subbereiche der Körperregion umfassen, die nicht durch einen Nutzer als diagnostisch relevante Subbereiche erfasst wurden. Beispielweise kann ein durch den Nutzer mittels der Eingabeoption als diagnostisch relevant gekennzeichneter Subbereich um einen oder mehrere Subbereiche der Körperregion ergänzt werden, der für die Bestimmung des Bildgebungsprotokolls notwendig ist. Wird mittels der Eingabeoption beispielsweise lediglich der Subbereich eines Zahns (oder mehrerer benachbarter Zähne) als diagnostisch relevanter Subbereich erfasst, können die benachbarten Subbereiche zu dem diagnostisch relevanten Subbereich hinzugefügt werden, um die Bilddaten des diagnostisch relevanten Subbereichs erfassen zu können. Insbesondere mittels einer Recheneinheit der Bildgebungsvorrichtung können Vorschriften oder Vorgaben zur Erstellung des Bildgebungsprotokolls für bestimmte Subbereiche von Körperregionen bei der Erfassung des diagnostisch relevanten Subbereich beachtet und/oder eingeschlossen werden.

Das Bestimmen des Bildgebungsprotokolls erfolgt in Abhängigkeit des erfassten diagnostisch relevanten Subbereichs. Das Bereitstellen des Bildprotokolls kann insbesondere in Abhängigkeit von Vorschriften oder Vorgaben zur Erstellung des Bildgebungsprotokolls für bestimmte Subbereiche von Körperregionen durch die Recheneinheit und/oder einen Algorithmus erfolgen. Das Bestimmen des Bildgebungsprotokolls kann insbesondere mindestens einen der folgenden Schritte umfassen: ein Auswählen eines oder mehrerer Bildgebungsprotokolle, ein Erstellen eines oder mehrerer Bildgebungsprotokolle, ein Auswählen eines oder mehrerer Bildgebungsparameter, ein Anpassen eines oder mehrerer Bildgebungsparameter, Erfassen einer Eingabe eines oder mehrerer Bildgebungsparameter durch einen Nutzer der Bildgebungsvorrichtung. Bevorzugt erfolgt das Bestimmen des Bildgebungsprotokolls ohne weitere Nutzerinteraktion in Abhängigkeit des diagnostisch relevanten Subbereichs. Beispielsweise kann anhand der Eingabeoption zum Erfassen der Information über Subbereich der Körperregion eine Anpassung eines Parameters des Bildgebungsprotokolls und/oder eine Anpassung eines Bildgebungsbereichs mittels einer Recheneinheit der Bildgebungsvorrichtung erfolgen.

Das Bereitstellen des Bildgebungsprotokolls kann einen finalen Vorbereitungsschritt einer Bildgebungsuntersuchung darstellen. Das Bildgebungsprotokoll kann insbesondere von einer Recheneinheit der Bildgebungsvorrichtung an eine Steuereinheit der Bildgebungsvorrichtung übermittelt werden, um die Bildgebungsuntersuchung in Abhängigkeit des Bildgebungsprotokolls durchzuführen.

In einer bevorzugten Ausführungsform umfasst das Bereitstellen der Eingabeoption zum Erfassen einer Information über einen Subbereich der Körperregion eine interaktive Auswahlmöglichkeit eines oder mehrerer diagnostisch relevanter Subbereiche. Das Bereitstellen der Eingabeoption an einen Nutzer erfolgt bevorzugt mittels einer Ausgabeeinheit, insbesondere einem Monitor. Das Erfassen des diagnostisch relevanten Subbereichs in Abhängigkeit der Eingabeoption stellt bevorzugt den einzigen Schritt und/oder Zeitpunkt in dem erfindungsgemäßen Verfahren zur Bestimmung des Bildgebungsprotokolls dar, welcher eine aktive Interaktion des Nutzers erfordert.

Das erfindungsgemäße Verfahren kann auf vorteilhafte Weise ein Erfassen einer Information über einen Subbereich einer Körperregion durch einen Nutzer der Bildgebungsvorrichtung in Abhängigkeit der bereitgestellten Eingabeoption ermöglichen. Dadurch kann auf vorteilhafte Weise eine Auswahl des diagnostisch relevanten Subbereichs der Körperregion unter Berücksichtigung sowohl von Vorschriften oder Vorgaben zur Erstellung des Bildgebungsprotokolls für bestimmte Subbereiche von Körperregionen und/oder Körperregionen als auch von individuellen Voraussetzungen einer anatomischen Struktur der Körperregion des Patienten ermöglicht werden.

Körperregionen von Patienten können sich individuell signifikant unterscheiden. Beispielsweise können Einflussgrößen wie eine Größe, ein Geschlecht und/oder ein Körperumfang eines Patienten eine Lage bestimmter Körperregionen bzw. eines Subbereichs einer Körperregion beeinflussen. Anatomische Strukturen der Körperregion des Patienten können jedoch, beispielweise aufgrund einer Erkrankungshistorie, auch gänzlich unterschiedliche Formen, Abmessungen oder räumliche Ausrichtungen aufweisen. Insbesondere anatomische Strukturen, welche sich in mehrere, annähernd ähnliche oder symmetrische Subbereiche untergliedern lassen, wie beispielsweise eine Hand oder eine Zahnregion eines Patienten, erfordern ein intensives Training von Nutzern der Bildgebungsvorrichtung, um ein Erfassen von Bilddaten eines gewünschten bzw. korrekten Subbereichs einer Körperregion und eine hohe Qualität der erfassten Bilddaten zu gewährleisten. Das erfindungsgemäße Verfahren ermöglicht dem Nutzer eine effiziente und assistierte Auswahl eines diagnostisch relevanten Subbereichs einer Körperregion zur automatisierten Bestimmung eines Bildgebungsprotokolls zur Erfassung von Bilddaten des Subbereichs der Körperregion des Patienten mittels einer Bildgebungsvorrichtung.

Insbesondere kann das vorgeschlagene Verfahren den Nutzer patientenspezifisch bei der Auswahl von Subbereichen einer Körperregion unterstützen. Dies kann insbesondere bei einer Bildgebungsuntersuchung von Zähnen, welche zwischen Patienten sehr unterschiedlich ausfallen können und wobei aufgrund eines hohen Maßes an Symmetrie zwischen verschiedenen Zahnabschnitten leicht Verwechselungen auftreten können, von Vorteil sein. Weiterhin kann das erfindungsgemäße Verfahren den Nutzer bei der Bestimmung eines Bildgebungsprotokolls in Abhängigkeit unterschiedlicher ausgewählter diagnostisch relevanter Subbereiche der Körperregionen des Patienten unterstützen. Beispielsweise kann dem Nutzer durch das Bereitstellen einer Eingabeoption der diagnostisch relevante Subbereich einer Körperregion unmittelbar ersichtlich sein und eine fachübliche Parametrierung des Bildgebungsprotokolls für den diagnostisch relevanten Subbereich automatisiert festgelegt werden. Der Aufwand, insbesondere für den Nutzer, zur Erstellung eines Bildgebungsprotokolls kann vorteilhafterweise erheblich reduziert, sowie der durchzuführende Workflow für einen Nutzer veranschaulicht und vereinfacht werden. Dadurch kann das vorgeschlagene Verfahren weiterhin auch einem Nutzer einer Bildgebungsvorrichtung ohne spezielle Vorkenntnisse ermöglichen, ein Bildgebungsprotokoll für einen diagnostisch relevanten Subbereich zu bestimmen. Insbesondere die Effizienz eines Prozesses des Erfassens des diagnostisch relevanten Subbereichs einer Körperregion kann mittels des Bereitstellens der Eingabeoption erhöht werden. Fehler bei dem Bestimmen eines Bildgebungsprotokolls und einem nachfolgenden Erfassen von weiteren Bilddaten des diagnostisch relevanten Subbereich einer Körperregion können somit vorteilhaft vermieden und ferner eine hohe Qualität der mittels der Bildgebungsvorrichtung erfassten Bilddaten des Subbereichs der Körperregion des Patienten gewährleisten werden.

In einer möglichen Ausführungsform des erfindungsgemäßen Verfahrens umfasst das Erfassen der Information über die Körperregion ein Erfassen von Patientendaten und/oder Bilddaten aus vorangegangenen Untersuchungen der Körperregion des Patienten und/oder einer Untersuchungsanweisung.

Das Erfassen der Information über die Körperregion des Patienten kann ein Abrufen oder Empfangen von Daten, insbesondere von Patientendaten, Bilddaten aus vorangegangenen Untersuchungen der Körperregion des Patienten und/oder Untersuchungsanweisungen umfassen. Die Information über die Körperregion kann beispielsweise von einer internen oder externen Speichereinheit und/oder einem medizinischen Informationssystem mittels einer Schnittstelle abgerufen werden. Ein medizinisches Informationssystem kann beispielsweise ein radiologisches Informationssystem (RIS) oder ein Krankenhaus-Informationssystem ("hospital information system" HIS) darstellen. Die Bildgebungsvorrichtung kann bevorzugt eine Steuereinheit und/oder eine Recheneinheit aufweisen, welche dazu ausgebildet sind, die Information über die Körperregion des Patienten mittels einer Schnittstelle von dem medizinischen Informationssystem, einer Cloud und/oder einer lokalen Speichereinheit zu beziehen.

Die Patientendaten können bevorzugt Information über den Patienten und/oder die Körperregion des Patienten, aber auch die Subbereiche der Körperregion des Patienten umfassen. Die Patientendaten können eine oder mehrere der folgenden Informationen umfassen: eine Eigenschaft und/oder Beschaffenheit des Patienten, wie Alter, Geschlecht, Gewicht und/oder Größe des Patienten, einen Befund und/oder eine Diagnose, insbesondere Informationen von in der Vergangenheit erfolgten Befundungen und/oder Behandlungen, eine beliebige andere medizinische und/oder demografische Information über den Patienten. Die Patientendaten können auch als Patienteninformation oder Patientenakte bezeichnet werden. Patientendaten können insbesondere in Form einer digitalen Akte und/oder Ansammlung von Informationen auf einem Speichermedium und/oder in einer Cloud-Anwendung vorliegen. Bevorzugt mittels eines Algorithmus, insbesondere eines Textverarbeitungsalgorithmus und/oder einer trainierten Funktion, kann die Information über die Körperregion aus den Patientendaten ermittelt werden.

Bilddaten aus vorangegangenen Untersuchungen der Körperregion des Patienten können auch als Untersuchungsbilddaten. Bilddaten aus vorangegangenen Untersuchungen der Körperregion des Patienten können ein oder mehrere Bilddatensätze aus ein oder mehr vorangegangenen Untersuchungen umfassen. Die Bilddaten können als Rohdaten vorliegen, bevorzugt jedoch als rekonstruierte Bilder und/oder verarbeitete Information, welche aus den Rohdaten abgeleitet werden. Bilddaten aus vorangegangenen Untersuchungen der Körperregion des Patienten können vorzugsweise Localizer-Bilddaten einer Localizer-Bildgebung umfassen. Eine Localizer-Bildgebung kann insbesondere eine zeiteffiziente Bildgebungsuntersuchung darstellen, bei welcher Localizer-Bilddaten der Körperregion des Patienten erfasst werden. Die Localizer-Bilddaten können beispielweise eine eingeschränkte bzw. reduzierte Qualität und/oder räumliche Auflösung im Vergleich zu Bilddaten einer konventionellen Bildgebungsuntersuchung aufweisen. Vorzugsweise stellt die Localizer-Bildgebung eine räumliche Auflösung bereit, welche für eine Detektion und/oder eine Identifizierung von anatomischen Strukturen, insbesondere von Subbereichen einer Körperregion wie z. B. einer Zahnregion, einem Zahnbogen, einem Kieferknochen oder dergleichen, geeignet ist. Insbesondere mittels eines Algorithmus, beispielsweise eines Bildverarbeitungsalgorithmus und/oder einer trainierten Funktion, können in Abhängigkeit der Bilddaten aus vorangegangenen Untersuchungen der Körperregion des Patienten, beispielsweise einer bereits in der Vergangenheit erfolgten Bildgebungsuntersuchung und/oder Localizer-Bildgebung, die Information über die Körperregion ermittelt werden.

Eine Untersuchungsanweisung kann insbesondere eine Information über einen durchzuführenden Bildgebungsvorgang und/oder eine durchzuführenden Bildgebungsuntersuchung umfassen. Beispielsweise kann eine Untersuchungsanweisung ein Ergebnis einer Anamnese und/oder Befundung eines Arztes in Form eines schriftlichen Untersuchungsberichts und/oder eine Überweisung an einen anderen Fachbereich und/oder Arzt umfassen. Eine Untersuchungsanweisung kann einen textuellen Bericht, eine schriftliche Anweisung, eine Markierung eines Arztes (beispielsweise einen segmentierten Bereich einer Körperregion) und/oder eine schriftliche Anmerkung eines Arztes und/oder eines medizinischen Fachpersonals umfassen. Insbesondere kann eine Untersuchungsanweisung Informationen zu einer Bildgebungsuntersuchung oder einer noch zu durchzuführenden Bildgebungsuntersuchung umfassen. Insbesondere kann eine Untersuchungsanweisung Informationen zu dem Patienten umfassen, die insbesondere nicht den Patientendaten zu entnehmen sind. Zudem können Untersuchungsanweisungen Informationen sein, die einer Person zugänglich gemacht werden können, die nicht der ärztlichen Schweigepflicht unterliegt. Insbesondere kann die Information über die Körperregion des Patienten und/oder mögliche relevante Subbereiche mittels eines Algorithmus oder einer trainierten Funktion, insbesondere zur Texterkennung und/oder-verarbeitung, in Abhängigkeit der Untersuchungsanweisung ermittelt werden.

Ein solcher Algorithmus kann insbesondere zur Verarbeitung der Patientendaten, Bilddaten aus vorangegangenen Untersuchungen und/oder Untersuchungsanweisungen ausgebildet sein. Beispielsweise kann der Algorithmus einen Logik-basierten Algorithmus, einen trainierten Algorithmus, einen selbstlernenden Algorithmus, ein künstliches neuronales Netzwerk, einen Machine-Learning Algorithmus und/oder einen Bildverarbeitungsalgorithmus umfassen. Das Erfassen der Information über die Körperregion eines Patienten kann insbesondere ein Erfassen einer Information aus Patientendaten, Bilddaten aus vorausgegangenen Untersuchungen und/oder einer Untersuchungsanweisung mittels des Algorithmus und/oder der trainierten Funktion umfassen.

In Abhängigkeit des Erfassens der Information über die Körperregion des Patienten kann insbesondere ein Bestimmen von Subbereichen der Körperregion erfolgen. Insbesondere kann eine Bestimmung der Subbereiche in Abhängigkeit einer Mehrzahl von Informationen über die Körperregion des Patienten erfolgen. Beispielweise kann eine Eingabeoption mit entsprechend bestimmten Subbereichen zur Auswahl durch einen Nutzer anhand der Patienteninformationen erzeugt werden. Insbesondere können die patientenspezifischen Informationen der Körperregion des Patienten durch eine trainierte Funktion und/oder einen Algorithmus extrahiert und auf Grundlage dessen eine Bestimmung der Subbereiche der Körperregion erfolgen.

Ein Erfassen der Information über die Körperregion in Abhängigkeit von Patientendaten, Bilddaten aus vorangegangenen Untersuchungen und/oder Untersuchungsanweisungen kann vorteilhaft eine für den Nutzer vereinfachte und/oder verbesserte Erfassung der Information über die Körperregion des Patienten ermöglichen. Insbesondere lässt sich durch eine Verarbeitung der genannten Informationen mittels eines Algorithmus ein Automatisieren des Erfassens der Körperregion des Patienten, aber auch ein Ermitteln der potenziell diagnostisch relevanten Subbereiche, ermöglichen. Somit können vorliegende technische Informationen über einen Patienten vorteilhaft zur Individualisierung und Adaption des Workflows einer Bestimmung eines Bildgebungsprotokolls für einen Nutzer eines Bildgebungsvorrichtung genutzt werden.

In einer möglichen Ausführungsform des erfindungsgemäßen Verfahrens umfasst das Ermitteln des Bildgebungsprotokolls zur Erfassung von Bilddaten des diagnostisch relevanten Subbereichs der Körperregion des Patienten in Abhängigkeit des erfassten diagnostisch relevanten Subbereichs ein Auswählen eines Bildgebungsprotokolls aus einer Mehrzahl von in einer Datenbank gespeicherten Bildgebungsprotokollen.

Beispielsweise können eine Steuereinheit und/oder eine Recheneinheit der Bildgebungsvorrichtung dazu ausgebildet sein, ein bestimmtes Bildgebungsprotokoll aus einer in einer Bibliothek oder einer Datenbank gespeicherten Mehrzahl von Bildgebungsprotokollen in Abhängigkeit des erfassten diagnostisch relevanten Subbereichs der Körperregion des Patienten auszuwählen und bereitzustellen. Bevorzugt können die Steuereinheit und/oder die Recheneinheit dazu ausgebildet sein, in Abhängigkeit der erfassten Information über die Körperregion für einen oder mehrere Subbereiche der Körperregion Bildgebungsprotokolle zu bestimmen und in einer Datenbank abzuspeichern. Es jedoch auch denkbar, dass eine manuelle Auswahl eines Bildgebungsprotokolls durch einen Nutzer aus einer Mehrzahl durch eine Speichereinheit und/oder Recheneinheit bereitstellte Bildgebungsprotokolle erfolgt. Mit anderen Worten kann durch die Steuereinheit und/oder die Recheneinheit ein Vorselektieren von Bildgebungsprotokollen erfolgen, insbesondere um einem Nutzer eine Auswahl eines Bildgebungsprotokolls zu erleichtern und/oder vorzuschlagen.

Eine Datenbank kann insbesondere ein Teil einer Speichereinheit der Bildgebungsvorrichtung sein, die dazu ausgebildet ist, ein oder mehrere Bildgebungsprotokolle zu speichern. Beispielweise kann eine Steuereinheit und/oder eine Recheneinheit der Bildgebungsvorrichtung eine solche Speichereinheit mit einer Datenbank umfassen. Für jeden möglichen durch eine Bildgebungsuntersuchung abbildbaren Subbereich einer Körperregion kann in der Datenbank ein parametriertes Bildgebungsprotokoll hinterlegt bzw. gespeichert sein. Die gespeicherten Bildgebungsprotokolle können umfassend oder nur teilweise parametriert sein. Insbesondere kann ein Bildgebungsbereich des diagnostisch relevanten Subbereichs durch ein parametriertes Bildgebungsprotokoll abgebildet und/oder vorbestimmt sein.

Ein Auswählen eines Bildgebungsprotokolls aus einer Mehrzahl von in einer Datenbank gespeicherten Bildgebungsprotokollen in Abhängigkeit des diagnostisch relevanten Subbereichs einer Körperregion kann vorteilhaft das Ermitteln eines für den diagnostisch relevanten Subbereich der Körperregion geeigneten Bildgebungsprotokolls ermöglichen. Durch das Parametrieren und Abspeichern von Bildgebungsprotokollen in einer Datenbank kann nach der Erfassung der Eingabe der Information über den Subbereich der Körperregion durch den Nutzer das Bildgebungsprotokoll zeiteffizient bereitstellt und/oder angepasst werden. Dies kann eine Reduzierung des Aufwands zur Erstellung eines Bildgebungsprotokolls und/oder eine Vereinfachung des Arbeitsablaufs zur Bestimmung eines Bildgebungsprotokolls ermöglichen.

In einer möglichen Ausführungsform des erfindungsgemäßen Verfahrens umfasst das Ermitteln des Bildgebungsprotokolls zur Erfassung von Bilddaten des diagnostisch relevanten Subbereichs der Körperregion des Patienten in Abhängigkeit des erfassten diagnostisch relevanten Subbereichs ein Bestimmen eines Bildgebungsparameters des Bildgebungsprotokolls.

Ein Bildgebungsprotokoll umfasst typischerweise mindestens einen Bildgebungsparameter. Ein Bildgebungsparameter kann ebenso als Parameter oder Bildgebungsprotokollparameter oder Protokollparameter bezeichnet werden. Durch ein Bildgebungsprotokoll kann ein Ablauf und eine Eigenschaft einer Bildgebungsuntersuchung, insbesondere von Bildgebungssequenzen einer Bildgebungsuntersuchung, festgelegt sein. Der mindestens eine Bildgebungsparameterkann eine Ansteuerung einer Komponente der Bildgebungsvorrichtung ermöglichen oder definieren. Bildgebungsparameter können beispielsweise Informationen über eine räumliche Auflösung, einen Kontrast, eine Schichtdicke, eine Abmessung eines Bildgebungsvolumens, eine Relaxationszeit, eine Echozeit und dergleichen umfassen. Ein Bildgebungsparameter kann insbesondere eine beliebige bildrelevante Einstellung der Bildgebungsvorrichtung, aber auch einen Parameter eines Workflows der Bildgebungsuntersuchung, umfassen. Insbesondere ein Bildgebungsbereich eines diagnostisch relevanten Subbereichs kann durch Bildgebungsparameter definiert sein.

Das Bestimmen eines Bildgebungsparameters des Bildgebungsprotokolls kann ein Auswählen eines Parameters (und/oder einer Gruppe von Parametern) aus einer Datenbank, ein Anpassen eines Parameters, aber auch ein Erfassen einer Eingabe eines Parameters umfassen. Das Auswählen eines Parameters und/oder einer Gruppe von Parametern kann beispielweise erfolgen, wenn ein teilparametriertes Bildgebungsprotokoll vorliegt. Bildgebungsparameter können bevorzugt in Abhängigkeit eines erfassten diagnostisch relevanten Subbereichs aus einer Datenbank ausgewählt und dem Bildgebungsprotokoll hinzugefügt werden. Beispielsweise kann ein Bildgebungsparameter für eine Körperregion für alle die den Körperbereich kennzeichnenden Subbereiche den gleichen Wert aufweisen, sodass der Bildgebungsparameter lediglich einmal in der Datenbank hinterlegt sein kann und in jedes Bildgebungsprotokoll eines Subbereichs übertragen werden kann. Das Auswählen eines Parameters aus einer Datenbank kann bevorzugt automatisch (bzw. ohne Nutzerinteraktion) erfolgen.

Das Anpassen eines Parameters kann ein Ändern und/oder Entfernen eines Parameters umfassen. Beispielsweise kann ein Wert eines Bildgebungsparameters eines vollständig parametrierten Bildgebungsprotokolls in Abhängigkeit des erfassten diagnostisch relevanten Subbereichs geändert werden. Beispielsweise kann aufgrund einer Patienteninformation die Anpassung eines Parameters des Bildgebungsprotokolls in Abhängigkeit des erfassten diagnostischen Subbereichs erfolgen.

Das Bestimmen eines Bildgebungsparameters kann das Erfassen einer Eingabe eines Parameters und/oder einer Änderung eines Parameters durch einen Nutzer umfassen. Eine Anzeigeeinheit, beispielsweise ein Monitor, kann zur Bereitstellung eines vollständig oder teilweise parametrierten Bildgebungsprotokolls ausgebildet sein. Die Anzeigeeinheit kann zudem eine Eingabeoption zur Anpassung eines Parameters eines Bildgebungsprotokolls bereitstellen. Beispielsweise können durch die Anzeigeeinheit nur die Bildgebungsparameter angezeigt werden, die einer Überprüfung und/oder einer Anpassung durch einen Nutzer bedürfen. Beispielsweise können für einen Bildgebungsparameter mehr als ein Wert in einer Datenbank hinterlegt sein, sodass durch einen Nutzer kann ein Wert ausgewählt werden kann.

Ein Bestimmen eines Bildgebungsparameters des Bildgebungsprotokolls kann ein gezieltes Anpassen eines oder mehrerer Parameter eines Bildgebungsprotokolls für einen diagnostisch relevanten Subbereich der Körperregion des Patienten ermöglichen. Durch die reine Auswahl eines Bildgebungsprotokolls aus einer Mehrzahl von in einer Datenbank gespeicherten Bildgebungsprotokollen können individuelle Voraussetzungen eines Patienten, insbesondere eines diagnostisch relevanten Subbereichs einer Körperregion des Patienten, nicht ausreichend berücksichtigt oder durch das Bildgebungsprotokoll des diagnostisch relevanten Subbereichs abgebildet sein. Vorteilhaft kann durch das Anpassen eines Bildgebungsparameters einem Nutzer das zeiteffiziente Anpassen eines Bildgebungsprotokolls ermöglicht werden. Dies kann eine Reduzierung des Aufwands zur Erstellung eines Bildgebungsprotokolls und/oder eine Vereinfachung des Arbeitsablaufs zur Bestimmung eines Bildgebungsprotokolls bedeuten.

In einer möglichen Ausführungsform des erfindungsgemäßen Verfahrens umfasst das Erfassen des diagnostisch relevanten Subbereichs in Abhängigkeit der Eingabeoption zum Erfassen der Information über den Subbereich der Körperregion ein Erfassen einer Mehrzahl diagnostisch relevanter Subbereiche. Das Ermitteln des Bildgebungsprotokolls zur Erfassung von Bilddaten des diagnostisch relevanten Subbereichs der Körperregion des Patienten in Abhängigkeit des diagnostisch relevanten Subbereichs umfasst ein Ermitteln eines Bildgebungsprotokolls für jeden der erfassten diagnostisch relevanten Subbereiche. Das Bereitstellen des Bildgebungsprotokolls für die Erfassung von Bilddaten des diagnostisch relevanten Subbereichs der Körperregion mittels der Bildgebungsvorrichtung umfasst eine Bereitstellung eines Bildgebungsprotokolls für jeden der erfassten diagnostisch relevanten Subbereiche.

Mit anderen Worten wird dem Nutzer bevorzugt eine Auswahl von mehr als einem diagnostisch relevanten Subbereich durch die Eingabeoption ermöglicht. Die diagnostisch relevanten Subbereiche können hierbei voneinander beabstandet sein, es können aber auch aneinander angrenzende und/oder benachbarte Subbereiche ausgewählt werden. Beabstandete diagnostisch relevante Subbereiche können insbesondere dadurch charakterisiert sein, dass ein oder mehrere Subbereiche zwischen den erfassten diagnostisch relevanten Subbereichen angeordnet sind.

Bevorzugt kann das Erfassen einer Mehrzahl diagnostisch relevanter Subbereiche ein Bestimmen der Positionierung und/oder Anordnung der erfassten diagnostisch relevanten Subbereiche umfassen. Anhand der Bestimmung der Positionierung und/oder Anordnung der erfassten diagnostisch relevanten Subbereiche kann ein Ermitteln des Bildgebungsprotokolls und ein Bereitstellen des Bildgebungsprotokolls für jeden der diagnostisch relevanten Subbereiche erfolgen. Insbesondere kann eine Priorisierung oder ein Bestimmen einer Reihenfolge der Bildgebungsuntersuchungen und/oder -sequenzen aus der Positionierung und/oder Anordnung der erfassten diagnostisch relevanten Subbereiche erfolgen. Eine Priorisierung, bzw. Reihenfolge der Bildgebungsuntersuchungen und/oder -sequenzen kann alternativ manuell durch einen Nutzer eingegeben werden. Beispielweise kann erfasst werden, in welcher Reihenfolge der Nutzer die diagnostisch relevanten Subbereiche ausgewählt hat und die Priorisierung, bzw. Abfolge der Bildgebungsuntersuchungen und/oder -sequenzen entsprechend erfolgen. Bevorzugt erfolgt bei dem Erfassen beabstandeter diagnostisch relevanter Subbereiche eine automatische Bestimmung des Bildgebungsprotokolls für jeden der erfassten diagnostisch relevanten Subbereiche. Dem Nutzer kann durch die Eingabeoption und/oder eine Ausgabe, bevorzugt interaktiv durch eine farbige Kodierung und/oder eine grafische Verdeutlichung, die Reihenfolge der Bildsequenzen aufgrund der ausgewählten Subbereiche verdeutlicht werden.

Das Erstellen eines Bildgebungsprotokolls für jeden der bestimmten diagnostisch relevanten Subbereiche kann einen vereinfachten Arbeitsablauf für den Nutzer ermöglichen. Dies kann eine zeiteffiziente und kosteneffiziente Bestimmung der Bildgebungsprotokolle bei gleichzeitiger Reduzierung von Fehlern (beispielsweise durch eine doppelte Bestimmung eines Bildgebungsprotokolls für einen Subbereich) ermöglichen. Besonders vorteilhaft ist dies bei Vorliegen einer komplex oder untypisch gestalteten Körperregion eines Patienten, beispielsweise einer Kieferregion, bei denen unerfahrene Nutzer leicht Fehler machen können.

In einer möglichen Ausführungsform des erfindungsgemäßen Verfahrens umfasst das Erfassen des diagnostisch relevanten Subbereichs in Abhängigkeit der Eingabeoption zum Erfassen der Information über einen Subbereich der Körperregion ein Erfassen einer Mehrzahl diagnostisch relevanter Subbereiche. Das Verfahren weist ferner ein Bestimmen eines gemeinsamen Bildgebungsbereichs der Körperregion anhand der Mehrzahl erfasster diagnostisch relevanter Subbereiche auf. Das Ermitteln des Bildgebungsprotokolls zur Erfassung von Bilddaten der diagnostisch relevanten Subbereiche der Körperregion des Patienten in Abhängigkeit des gemeinsamen Bildgebungsbereichs erfolgt.

Mit anderen Worten ist dem Nutzer bevorzugt eine Auswahl von mehr als einem Subbereich, beispielweise zweier Subbereiche der Körperregion, durch die Eingabeoption möglich. Die Subbereiche können hierbei insbesondere aneinander angrenzen und/oder benachbart sein, es können aber auch voneinander beanstandete Subbereiche ausgewählt werden. Die Subbereiche können sich überlappen und/oder einander einschließen.

Bevorzugt kann das Erfassen einer Mehrzahl diagnostisch relevanter Subbereiche ein Bestimmen der Positionierung und/oder Anordnung der erfassten diagnostisch relevanten Subbereiche umfassen. Durch die Bestimmung der Positionierung und/oder Anordnung der erfassten diagnostisch relevanten Subbereiche kann insbesondere bestimmbar sein, ob die Ermittlung des Bildgebungsprotokolls und die Bereitstellung des Bildgebungsprotokolls mittels eines gemeinsamen Bildgebungsprotokoll der Subbereiche erfolgen kann. Bei einer bestimmten benachbarten Anordnung der erfassten Subbereiche kann insbesondere ein Bestimmen eines gemeinsamen Bildgebungsbereichs der Körperregion erfolgen. Die Bestimmung des gemeinsamen Bildgebungsbereichs der Körperregion kann insbesondere in Abhängigkeit der zwei oder mehr erfassten Subbereiche der Körperregionen erfolgen. Überlappen sich beispielsweise zwei der erfassten Subbereiche einer Körperregion, kann insbesondere ein gemeinsamer Bildgebungsbereich bestimmt werden, um eine wiederholte Aufnahme eines Subbereichs einer Körperregion eines Patienten zu vermeiden.

Der gemeinsame Bildgebungsbereich kann vorzugsweise einen oder mehrere diagnostisch relevante Subbereiche der Körperregion des Patienten umfassen oder abdecken. Insbesondere kann der gemeinsame Bildgebungsbereich durch ein Volumen charakterisiert sein kann, das die erfasste Mehrzahl der diagnostisch relevanten Subbereiche einschließt. Insbesondere kann der gemeinsame Bildgebungsbereich ein Volumen innerhalb eines Patientenaufnahmebereichs der Bildgebungsvorrichtung definieren, aus welchem Bilddaten mittels einer Bildgebungsuntersuchung erfasst werden. Vorzugsweise sind ein oder mehrere diagnostisch relevante Subbereiche der Körperregion des Patienten für eine Bildgebungsuntersuchung in einem sogenannten Bildgebungsvolumen der Bildgebungsvorrichtung positioniert. Ein Parameter des gemeinsamen Bildgebungsbereichs kann beispielsweise eine räumliche Position, eine Ausrichtung, eine Dimension, und/oder eine Form des gemeinsamen Bildgebungsbereichs definieren. Der gemeinsame Bildgebungsbereich kann insbesondere ein Messvolumen, ein Field-of-View, ein Sichtfenster oder eine Schichtausrichtung umfassen.

Die Eingabeoption kann, insbesondere bei einer Auswahl einer Mehrzahl von diagnostisch relevanten Subbereichen, eine grafische Repräsentation des gemeinsamen Bildgebungsbereichs umfassen, um dem Nutzer den gemeinsamen Bildgebungsbereich einer Bildgebungsvorrichtung zu verdeutlichen. Die Eingabeoption kann eine Ausgabe einer Information über eine empfohlene und/oder ideale Auswahl eines oder mehrerer Subbereiche und/oder über eine ideale Positionierung des gemeinsamen Bildgebungsbereichs mittels Ausgabeeinheit einer Benutzerschnittstelle umfassen. Beispielweise können eine Steuereinheit und/oder eine Recheneinheit der Bildgebungsvorrichtung dazu ausgebildet sein, eine bestimmte Information über eine gewünschte oder ideale Einstellung des gemeinsamen Bildgebungsbereichs aus einer Bibliothek oder einer Datenbank in Abhängigkeit der Körperregion des Patienten auszuwählen und bereitzustellen. Aufgrund dieser Information kann es dem Nutzer bevorzugt ermöglicht werden, eine ideale Auswahl von diagnostisch relevanten Subbereichen vorzunehmen.

Die vereinfachte Bestimmung eines Bildgebungsprotokolls für einen gemeinsamen Bildgebungsbereich der bestimmten diagnostisch relevanten und vorzugsweise benachbart angeordneten Subbereiche kann eine Reduzierung einer Anzahl von durchzuführenden Bildgebungsuntersuchungen ermöglichen. Durch das assistierte Bestimmen des gemeinsamen Bildgebungsbereichs bzw. eines Bildgebungsprotokolls für den gemeinsamen Bildgebungsbereich lässt sich ein zeiteffizienter und kosteneffizienter Arbeitsablauf zur Bestimmung des Bildgebungsprotokolls bei gleichzeitiger Reduzierung von manuellen Eingaben durch einen Nutzer ermöglichen. Besonders vorteilhaft ist dies bei Vorliegen einer komplex oder untypisch gestalteten Körperregion eines Patienten, beispielsweise einer Kieferregion, für die eine Erstellung eines Bildgebungsprotokolls durch einen unerfahrenen Nutzer typischerweise nicht durchführbar sein können.

In einer möglichen Ausführungsform umfasst das erfindungsgemäße Verfahren ferner ein Bereitstellen einer weiteren Eingabeoption zum Erfassen einer weiteren Information über den diagnostisch relevanten Subbereich. Ferner durch das erfindungsgemäße Verfahren umfasst ist ein Erfassen einer Eingabe in Abhängigkeit der Eingabeoption zum Erfassen der weiteren Information über den diagnostisch relevanten Subbereich. Das Ermitteln des Bildgebungsprotokolls zur Erfassung von Bilddaten des diagnostisch relevanten Subbereichs der Körperregion des Patienten erfolgt zusätzlich in Abhängigkeit der Information.

Durch die weitere Eingabeoption kann dem Nutzer eine Eingabe einer weiteren Information über einen Subbereich der Körperregion ermöglicht werden, die zur Bestimmung des Bildgebungsprotokolls für den Subbereich der Körperregion notwendig und/oder zweckmäßig ist. Insbesondere kann die weitere Eingabeoption eine grafische Repräsentation eines Subbereichs einer Körperregion und/oder eine textbasierte Eingabeoption umfassen. Durch eine textbasierte Eingabeoption kann es dem Nutzer ermöglicht werden, beispielweise Stichworte oder Kurzinformationen einzugeben, anhand derer das Bildgebungsprotokoll ermittelt wird. Es ist vorstellbar, dass das Ermitteln des Bildgebungsprotokolls mittels eines Algorithmus, insbesondere einer trainierten Funktion, erfolgt.

Die weitere Eingabeoption ist vorzugsweise optional. Das Verfahren kann beispielsweise in Anwendungsfällen mit komplexer anatomischer Struktur der Körperregion eine weitere Eingabeoption umfassen. Bei der Bestimmung des Bildgebungsprotokolls für einen Subbereich einer Körperregion eines Patienten kann insbesondere der Fall eintreten, dass mehr als eine Parametrierungsoption für das Bildgebungsprotokoll vorliegt bzw. in einer Datenbank hinterlegt ist. In diesem Fall kann einem Nutzer durch die weitere Eingabeoption eine Auswahl einer der Optionen ermöglicht werden. Bevorzugt umfasst das Verfahren lediglich eine weitere Eingabeoption, es ist jedoch denkbar, dass eine beliebige Mehrzahl weiterer Eingabeoptionen bereitgestellt werden und eine Mehrzahl weiterer Informationen in Abhängigkeit der Mehrzahl von Eingabeoptionen erfasst werden. Das Ermitteln des Bildgebungsprotokolls kann in Abhängigkeit der Mehrzahl von weiteren Informationen erfolgen.

Eine weitere Eingabeoption kann den Arbeitsablauf der Bildgebungsprotokollbestimmung für einen Subbereich einer Körperregion vereinfachen. Insbesondere in Anwendungsfällen, in denen eine Bestimmung des Bildgebungsprotokolls nicht ohne weiteren Nutzerinput möglich ist, kann die weitere Eingabeoption sicherstellen, dass eine zeiteffiziente Erfassung der notwendigen Informationen zur Bildgebungsprotokollbestimmung möglich ist. Zudem kann durch eine weitere Eingabeoption insbesondere einem erfahrenen Nutzer eine weitere Spezifizierungsmöglichkeit des Bildgebungsprotokolls ermöglicht werden. Eine weitere Eingabeoption kann daher die Flexibilität bezüglich der möglichen Anwendungsfälle, insbesondere bezüglich der abbildbaren Körperregionen und/oder Subbereiche von Körperregionen, erhöhen.

In einer möglichen Ausführungsform des erfindungsgemäßen Verfahrens umfasst die weitere Eingabeoption zum Erfassen der weiteren Information über den diagnostisch relevanten Subbereich ein Erfassen eines der folgenden Informationen: einen Bildgebungsparameter des Bildgebungsprotokolls und/oder eine Anzahl von Bildgebungsuntersuchungen und/oder eine Reihenfolge von Bildgebungsuntersuchungen und/oder einen Teilbereich des Subbereichs der Körperregion des Patienten.

Bei der Parametrierung eines Bildgebungsprotokolls für einen Subbereich der Körperregion kann ein Bildgebungsparameter des Bildgebungsprotokolls unbekannt oder nicht in eindeutiger Weise parametrierbar sein. Beispielweise kann in einer Datenbank keine Information zu dem Bildgebungsparameter gespeichert sein. Beispielweise können aber auch mehrere Informationen und/oder Optionen für einen Bildgebungsparameter in einer Datenbank gespeichert sein. Bespielweise kann aber auch aufgrund einer Auswahl einer Mehrzahl von diagnostisch relevanten Subbereichen eine Information über einen Bildgebungsparameter unbekannt sein und/oder mehrere Parametrierungsoptionen vorliegen. Die weitere Eingabeoption kann die Eingabe und Erfassung einer weiteren Information bezüglich eines Bildgebungsparameters durch einen Nutzer ermöglichen. Die weitere Eingabeoption kann insbesondere in Form eines Fensters ausgestaltet sein, welches einem Nutzer mittels einer Benutzerschnittstelle nur dann bereitgestellt wird, wenn ein Bildgebungsparameter unbekannt ist oder mehrere Parametrierungsoptionen für den Bildgebungsparameter ermittelt worden sind. Das Fenster kann einem Nutzer eine Texteingabe der Information des Bildgebungsparameters und/oder mögliche Parametrierungsoptionen anzeigen und/oder eine Auswahl einer Parametrierungsoptionen mittels der Benutzeroberfläche ermöglichen. Die weitere Eingabeoption kann beispielweise ebenso als Sprach-Abfrage ausgestaltet sein, die dem Nutzer vorzugsweise durch eine Sprachantwort eine Eingabe und/oder Auswahl ermöglicht.

Insbesondere bei einer Auswahl einer Mehrzahl von diagnostisch relevanten Subbereichen durch einen Nutzer kann eine Information über eine Anzahl von Bildgebungsuntersuchungen benötigt werden. Die weitere Eingabeoption kann insbesondere in Form eines Fensters und/oder eines Hinweises ausgestaltet sein, welches dem Nutzer nur angezeigt wird, wenn eine Mehrzahl von Subbereichen ausgewählt sind. Die weitere Eingabeoption kann auch eine Abfrage vor dem Erfassen der Information über einen (oder mehrere) Subbereiche der Körperregion umfassen. Die Abfrage kann beispielweise eine Auswahlmöglichkeit darstellen, ob eine oder mehrere Bildgebungsuntersuchungen zulässig und/oder durch einen Nutzer gewünscht sind. Beispielweise kann die Eingabeoption einen Schwellwert bzw. Maximalwert von zulässigen Bildgebungsuntersuchungen umfassen. Einem Nutzer ist hierdurch beispielweise eine Auswahl zwischen einer zeiteffizienten ersten Option (z. B. lediglich eine zulässige Bildgebungsuntersuchung) oder einer zweiten Option mit detaillierten Bildaufnahmen (z. B. eine Mehrzahl oder eine bestimmte Anzahl von Bildgebungsuntersuchungen) möglich.

Insbesondere bei einer Auswahl einer Mehrzahl von diagnostisch relevanten Subbereichen durch einen Nutzer kann die Bestimmung einer Mehrzahl von Bildgebungsprotokollen für eine Mehrzahl von Bildgebungsuntersuchungen erfolgen. In diesem Fall kann durch eine weitere Eingabeoption die Reihenfolge der Bildgebungsuntersuchungen festlegbar sein. Dies kann vorteilhaft sein, da durch eine Sondierung von bereits erfolgten Bildgebungsuntersuchungen während einer Abfolge von Bildgebungsuntersuchungen noch nicht erfolgte Bildgebungsuntersuchungen als nicht mehr notwendig klassifiziert und/oder abgebrochen werden können. Eine unnötige Verlängerung der Dauer der Bildgebungsuntersuchung und/oder eine weitere Belastung des Patienten durch eine nicht mehr notwendige Bildgebungsuntersuchung kann somit vorteilhaft vermieden werden. In anderen Anwendungsfällen kann eine erste zeitlich begrenzte Bildgebungsuntersuchung mit geringer Bildqualität erfolgen, um weitere Befundungen zu ermöglichen und/oder die weiteren Behandlungen des Patienten zu planen. Eine grafische, beispielweise in Form eines Zeitstrahls, oder textuelle Repräsentation, beispielweise in Form einer Tabelle, der Abfolge der Bildgebungsprotokolle kann durch die weitere Eingabeoption umfasst sein. Dem Nutzer kann beispielweise eine grafische Sortierung und/oder Priorisierung der Bildgebungsprotokolle und/oder die textuelle Eingabe einer Abfolge ermöglicht werden.

Die weitere Eingabeoption kann ähnlich der ersten Eingabeoption dem Nutzer eine Auswahl eines Subbereichs einer Körperregion bzw. eines Teilbereichs eines Subbereichs ermöglichen. Beispielsweise kann der Nutzer durch die erste Eingabeoption einen Subbereich einer Körperregion auswählen, der jedoch in weitere Teilbereiche untergliedert ist. Die weitere Eingabeoption kann dem Nutzer auch eine Auswahl eines oder mehrerer Teilbereiche des Subbereichs ermöglichen. Beispielsweise kann durch die erste Eingabeoption die Bestimmung der Subbereiche der oberen und unteren Kieferknochen einer Kieferregion eines Patienten als diagnostisch relevante Subbereiche durch einen Nutzer erfolgt sein. Zudem kann jeweils eine zweite Eingabeoption, beispielweise zur Auswahl eines Teilbereichs des vorderen die Schneidezähne beinhaltenden Knochenbereichs, für den jeweiligen Subbereich der Kieferregion des Patienten bereitgestellt werden.

Das Erfassen der genannten Informationen mittels einer weiteren Eingabeoption kann eine weitere Individualisierung bzw. patientenspezifische Anpassung des Bildgebungsprotokolls ermöglichen. Dies kann sowohl eine vorteilhafte Bildgebungsuntersuchung für den Patienten, insbesondere durch eine Reduzierung der Untersuchungsbelastung, als auch einen flexiblen Ablauf von Bildgebungsuntersuchungen für ärztliches Personal ermöglichen. Insbesondere ist durch eine weitere Eingabeoption der Untersuchungsprozess des Patienten und/oder der Arbeitsablauf der Bestimmung eines Bildgebungsprotokolls im Allgemeinen optimierbar.

In einer möglichen Ausführungsform des erfindungsgemäßen Verfahrens umfasst die weitere Eingabeoption zum Erfassen der weiteren Information über den diagnostisch relevanten Subbereich ein Ermitteln eines vorläufigen Bildgebungsbereichs. Der vorläufige Bildgebungsbereich ist an den diagnostisch relevanten Subbereich angepasst.

Das Ermitteln eines vorläufigen Bildgebungsbereichs kann beispielsweise ein Bestimmen des vorläufigen Bildgebungsbereichs, ein Bereitstellen des vorläufigen Bildgebungsbereichs und/oder ein Erfassen einer Korrektur des vorläufigen Bildgebungsbereichs durch einen Nutzer umfassen. Die Korrektur des vorläufigen Bildgebungsbereichs kann insbesondere durch die weitere Eingabeoption zum Erfassen einer weiteren Information über den diagnostisch relevanten Subbereich, im Folgenden als Subbereich bezeichnet, erfolgen.

Das Bestimmen des vorläufigen Bildgebungsbereichs kann ein Bestimmen einer Dimension, einer räumlichen Anordnung und/oder einer räumlichen Ausrichtung des vorläufigen Bildgebungsbereichs relativ zu der Körperregion des Patienten, insbesondere den erfassten diagnostisch relevanten Subbereichen einer Körperregion, umfassen. Das Bestimmen des vorläufigen Bildgebungsbereichs kann insbesondere durch einen Algorithmus, insbesondere eine trainierte Funktion, erfolgen.

Vorzugsweise wird eine, insbesondere grafische, Repräsentation des vorläufigen Bildgebungsbereichs durch das Bereitstellen der weiteren Eingabeoption an einen Nutzer, insbesondere mittels einer Benutzerschnittstelle, bereitgestellt und/oder angezeigt. Die Repräsentation des vorläufigen Bildgebungsbereichs kann beispielsweise ein Symbol, ein grafisches Objekt und/oder eine grafische Darstellung des vorläufigen Bildgebungsbereichs umfassen. Die Repräsentation des vorläufigen Bildgebungsbereichs kann beispielsweise ein Fenster mit einem ovalen oder polygonalen Rahmen umfassen. Insbesondere kann das Bereitstellen der Repräsentation des vorläufigen Bildgebungsbereichs eine Überlagerung der Repräsentation des vorläufigen Bildgebungsbereichs mit der Repräsentation der Körperregion und/oder der Subbereiche der Körperregion umfassen.

Bevorzugt wird ein vorläufiger Bildgebungsgereich einem Nutzer vorgeschlagen und eine Korrektur und/oder Anpassung des vorläufigen Bildgebungsbereichs durch die weitere Eingabeoption ermöglicht. Der vorläufige Bildgebungsbereich kann dem Bildgebungsbereich entsprechen. Die Repräsentation des vorläufigen Bildgebungsbereichs kann einem Nutzer durch Verschieben des vorläufigen Bildgebungsbereichs eine Anpassung des vorläufigen Bildgebungsbereichs ermöglichen. Beispielsweise kann ein Polygon in einer grafischen Repräsentation einer Körperregion verschoben werden, sodass ein erfasster Subbereich vollständig und ein weiterer erfasster Subbereich teilweise von dem vorläufigen Bildgebungsbereich umfasst sind. Beispielsweise kann aber auch die Orientierung eines mehrere Subbereiche umfassenden Polygons in einer grafischen Repräsentation durch einen Nutzer angepasst werden, um insbesondere den Aufnahmewinkel einer Bildgebungsuntersuchung anzupassen. Die Ausgabe der grafischen Repräsentation des vorläufigen Bildgebungsbereich an einen Nutzer und die Anpassung des vorläufigen Bildgebungsbereichs durch den Nutzer werden vorzugsweise durch eine Ausgabeeinheit und/oder eine Benutzerschnittstelle der Bildgebungsvorrichtung ermöglicht.

Dem Nutzer kann durch die weitere Eingabeoption eine Anpassung des vorläufigen Bildgebungsbereichs ermöglich werden. Ein durch den Nutzer angepasster Bildgebungsbereich kann die Qualität und/oder Effizienz einer mittels des Bildgebungsprotokolls durchgeführten Bildgebungsuntersuchung erhöhen. Insbesondere bei komplexen Körperregionen und/oder erfassten Subbereichen kann eine interaktive Anpassungsmöglichkeit des vorläufigen Bildgebungsbereich das Verfahren der Bestimmung eines Bildgebungsprotokolls für einen Nutzer vorteilhaft verdeutlichen und/oder vereinfachen.

In einer möglichen Ausführungsform des erfindungsgemäßen Verfahrens umfasst das Bereitstellen der Eingabeoption zum Erfassen der Information über den Subbereich der Körperregion in Abhängigkeit der Information über die Körperregion ein Bereitstellen einer Eingabeoption durch eine grafische Repräsentation der Körperregion und/oder eine grafische Auswahl des Subbereichs der Körperregion und/oder eine textbasierte Auswahl des Subbereichs der Körperregion.

Eine grafische Repräsentation der Körperregion kann eine piktografische, schematische und/oder bildhafte Information über einen Körperbereich umfassen. Es ist ebenso vorstellbar, dass die grafische Repräsentation der Körperregion eine piktografische, schematische und/oder bildhafte Information über die Subbereiche der Körperregion umfasst. Die grafische Repräsentation kann eine Information über einen gewünschten und/oder idealen Bildgebungsparameter für ein Bildgebungsprotokoll und/oder eine Hilfestellung und/oder (grafische) Anleitung zur Auswahl eines diagnostisch relevanten Subbereichs der Körperregion durch den Nutzer umfassen.

Durch eine grafische Auswahl des Subbereichs der Körperregion kann dem Nutzer ein auszuwählender diagnostisch relevanter Subbereich vorgeschlagen werden. Durch eine grafische Auswahl des Subbereichs der Körperregion kann dem Nutzer eine Anwahl eines Subbereichs mittels einer Benutzerschnittstelle, beispielsweise mittels eines Erfassens einer Auswahl eines Subbereichs in einer grafischen Repräsentation der Körperregion ermöglicht werden. Es ist ebenso vorstellbar eine derartige Auswahl mittels Anklickens des Subbereichs in der grafischen Repräsentation in einer mittels der Benutzerschnittstelle bereitgestellten Benutzeroberfläche zu ermöglichen. Vorzugsweise umfasst die grafische Auswahl des Subbereichs der Körperregion eine farbliche Markierung und/oder Umrandung eines Subbereichs der Körperregion.

Durch das Bereitstellen einer textbasierten und/oder einer grafischen Auswahl des Subbereich und/oder einer grafischen Repräsentation einer Körperregion können auch unerfahrene Nutzer über eine fachübliche Parametrierung des Bildgebungsprotokolls in Abhängigkeit des diagnostisch relevanten Subbereichs der Körperregion informiert werden. Dadurch lassen sich Kosten für ein Training des Personals, aber auch Fehler bei der Bestimmung des Bildgebungsprotokolls, auf vorteilhafte Weise reduzieren.

In einer möglichen Ausführungsform des erfindungsgemäßen Verfahrens umfasst die grafische Repräsentation der Körperregion und/oder die grafische Auswahl des Subbereichs der Körperregion der Eingabeoption eine zweidimensionale Repräsentation der Körperregion und/oder der Subbereiche der Körperregion. Insbesondere kann die zweidimensionale Repräsentation eine Schnittrepräsentation darstellen.

Die zweidimensionale Repräsentation der Körperregion kann eine vereinfachte und/oder schematisierte Darstellung der Körperregion umfassen. Die zweidimensionale Repräsentation kann eine vereinfachte und/oder schematisierte Schnittrepräsentation einer Körperregion umfassen. Insbesondere kann die zweidimensionale Repräsentation zwei Schnittrepräsentationen der Körperregion des Patienten umfassen, die bevorzugt entlang unterschiedlicher, vorzugsweise orthogonal zueinander ausgerichteter, Bezugsebenen ausgerichtet sind. Die Bezugsebenen der Schnittrepräsentation können die Sagittalebene, die Frontalebene oder die Transversalebene des Patienten umfassen. Beispielweise kann die zweidimensionale Repräsentation der Körperregion eine vertikal übereinander angeordnete Repräsentation, insbesondere mittels/in einer Benutzerschnittstelle, des unteren und oberen Kieferknochenbereichs eines Patienten mit der Transversalebene des Patienten als Spiegelebene umfassen. Es ist ebenso vorstellbar, dass das die zweidimensionale Repräsentation der Körperregion mehrere Repräsentation mit unterschiedlichen Bezugsebenen umfasst, beispielsweise einen Kieferknochenbereichs eines Patienten mit der Transversalebene und der Sagittalebene als Bezugsebenen.

Ein Bereitstellen einer zweidimensionalen Repräsentation der Körperregion und/oder der Subbereiche der Körperregion kann eine Veranschaulichung der Körperregion und/oder der Subbereiche in zumindest einer Bezugsebene erlauben. Dadurch kann es einem Nutzer der Bildgebungsvorrichtung auf vorteilhafte Weise ermöglicht werden, die Anordnung der Subbereiche der Körperregion unter Berücksichtigung einer individuellen Gestalt der Körperregion des Patienten zu erkennen und entsprechend eine Auswahl des diagnostisch relevanten Subbereichs vorzunehmen. Gerade bei der Auswahl von Subbereichen in einer Kieferregion mit vielen symmetrischen Subbereichen/Regionen kann dies die Auswahl vereinfachen. Insbesondere eine schematische zweidimensionale Repräsentation der Körperregion und/oder der Subbereiche der Körperregion kann zu einer vereinfachten Erkennung eines diagnostisch relevanten Subbereichs durch einen Nutzer führen, da trotz Berücksichtigung der individuellen Gestalt des Patienten die Erkennung anhand einer in der Regel für die meisten Patienten ähnlichen schematischen Repräsentation erfolgen kann.

In einer möglichen Ausführungsform des Verfahrens umfasst die Körperregion des Patienten eine Kieferregion und/oder eine Zahnregion.

Beispielsweise umfassen die Kieferregion und/oder die Zahnregion ein Gebiss, einen Abschnitt eines Kieferknochens, einen Abschnitt eines Gebisses, einen oder mehrere Zahnbögen, einen Abschnitt eines Zahnbogens, eine Gingiva, einen Abschnitt einer Gingiva und/oder einen oder mehrere Zähne des Patienten.

Eine Zahnregion eines Patienten weist zwei im Wesentlichen gleichförmige Zahnbögen auf, welche wiederum Abschnitte auf der linken Körperhälfte des Patienten und der rechten Körperhälfte des Patienten aufweisen. Zudem sind Reihen von Frontzähnen der Zahnbögen unter einem Winkel zu Reihen von Backenzähnen der Zahnbögen angeordnet, weshalb zahlreiche Abschnitte der Zahnregion bei der Bildgebungsuntersuchung der Zahnregion unterschieden werden müssen. Die Subbereiche einer Kieferregion und/oder eine Zahnregion können insbesondere für einen ungeübten Benutzer optisch sehr ähnlich sein, wodurch ein Risiko einer Verwechslung einer rechten Seite mit einer linken Seite eines Zahnbogens oder eines Abschnitts eines unteren Zahnbogens mit einem Abschnitt eines oberen Zahnbogens besteht.

Durch das Bereitstellen einer Eingabeoption zum Erfassen einer Information über einen Subbereich der Körperregion in Abhängigkeit der Information über die Körperregion kann ein direkter Bezug zu einem für die Bildgebungsuntersuchung relevanten Subbereich der Körperregion hergestellt werden. Dadurch lässt sich ein Risiko eines Auswählens eines falschen Subbereichs der Körperregion durch einen Nutzer der Bildgebungsvorrichtung auch bei komplexen anatomischen Strukturen, wie einer Kieferregion und/oder eine Zahnregion mit unterschiedlichen Abschnitten, auf vorteilhafte Weise reduzieren oder vermeiden.

In einer bevorzugten Ausführungsform umfasst die Erfassung von Bilddaten eines diagnostisch relevanten Subbereichs eine oder mehrere Magnetresonanzuntersuchungen einer Kieferregion oder einer Zahnregion eines Patienten. Es ist vorstellbar, dass eine Bildgebungssequenz von zumindest einer Magnetresonanzuntersuchung eine sehr kurze Echozeit aufweist, um eine kurze T2-Relaxationszeit von Spins eines Dentins oder eines Zahnschmelzes eines Zahns zu kompensieren und diese Bereiche mit hoher Signalintensität in erfassten Bilddaten darzustellen. Sehr kurze Echozeiten können kleiner als 150 µs oder kleiner als 70 µs sein. Mögliche Bildgebungssequenzen stellen beispielsweise FLASH ("fast low-angle shot") oder UTE ("ultra-short echo time") Sequenzen dar. Es ist jedoch ebenso vorstellbar, dass Bildgebungssequenzen mit einer längeren Echozeit, wie z. B. eine TSE ("turbo spin echo") Sequenz, verwendet werden. Bei solchen Sequenzen kann ein Erfassen des Magnetresonanzsignals des Zahnschmelzes oder des Dentins vermieden werden. In Bilddaten solcher Bildgebungssequenzen lassen sich die Zähne durch einen Mangel an Signalintensität im Vergleich zu einem umliegenden Gewebe differenzieren.

In einer bevorzugten Ausführungsform des erfindungsgemäßen Verfahrens umfasst das Erfassen der Information über die Körperregion des Patienten ein Erfassen eines Abschnitts einer Zahnregion, insbesondere eines Abschnitts eines oder mehrerer Zahnbögen, des Patienten.

In einer bevorzugten Ausführungsform des erfindungsgemäßen Verfahrens erfolgt das Erfassen von Bilddaten der Körperregion mittels eines Antennenelements oder einer Mehrzahl von Antennenelementen einer Magnetresonanzvorrichtung. Die Antennenelemente können hierfür beispielsweise an einer Kieferregion und/oder in einer Mundhöhle des Patienten positioniert sein. Das Antennenelement oder die Mehrzahl von Antennenelementen können insbesondere dazu ausgelegt sein, Magnetresonanzsignale der Kieferregion zu empfangen und diese an eine Empfangseinheit der Magnetresonanzvorrichtung zu übermitteln.

Die erfindungsgemäße Bildgebungsvorrichtung ist zur Erfassung von Bilddaten eines diagnostisch relevanten Subbereichs einer Körperregion eines Patienten ausgebildet. Die Bildgebungsvorrichtung umfasst eine Steuereinheit, eine Ausgabeeinheit und eine Benutzerschnittstelle. Die Steuereinheit ist dazu ausgebildet, ein Verfahren gemäß einer oben beschriebenen Ausführungsformen mittels der Bildgebungsvorrichtung auszuführen. Die Ausgabeeinheit ist dazu ausgebildet, einem Nutzer der Bildgebungsvorrichtung die Eingabeoption zum Erfassen der Information über den Subbereich der Körperregion bereitzustellen. Die Benutzerschnittstelle ist dazu ausgebildet, das Bildgebungsprotokoll in Abhängigkeit des diagnostisch relevanten Subbereichs bereitzustellen.

Die Steuereinheit kann dazu ausgebildet sein, das Bildgebungsprotokoll in Abhängigkeit des diagnostisch relevanten Subbereichs zu ermitteln. Die Benutzerschnittstelle kann dazu ausgebildet sein einem Nutzer die Auswahl eines Bildgebungsprotokoll in Abhängigkeit des diagnostisch relevanten Subbereichs zu ermöglichen.

In einer bevorzugten Ausführungsform ist die erfindungsgemäße Bildgebungsvorrichtung als eine Magnetresonanzvorrichtung ausgestaltet. Bevorzugt kann die Magnetresonanzvorrichtung kann dazu ausgebildet sein, Magnetresonanzdaten, insbesondere Magnetresonanzbilder, eines in einem Patientenaufnahmebereich der Magnetresonanzvorrichtung positionierten Patienten zu erfassen. Die Magnetresonanzdaten oder Magnetresonanzbilddaten können Bilddaten gemäß einer oben beschriebenen Ausführungsform umfassen.

Die erfindungsgemäße Magnetresonanzvorrichtung ist dazu ausgebildet, eine oder mehrere Bildgebungsuntersuchungen eines und/oder mehrerer Subbereiche der Körperregion des Patienten gemäß einer oben beschriebenen Ausführungsform des vorgeschlagenen Verfahrens durchzuführen. Durch die Verwendung einer Magnetresonanzvorrichtung kann eine Belastung des Patienten mit ionisierender Strahlung im Vergleich zu Röntgengeräten oder Computertomografie-Geräten auf vorteilhafte Weise vermieden werden.

Die Steuereinheit kann bevorzugt dazu ausgebildet sein, ein Verfahren gemäß einer oben beschriebenen Ausführungsformen zu koordinieren und mittels der Bildgebungsvorrichtung auszuführen. Die Steuereinheit kann bevorzugt in die Bildgebungsvorrichtung integriert sein oder als eine eigenständige Komponente ausgestaltet sein.

Die Ausgabeeinheit ist bevorzugt dazu ausgebildet, einem Nutzer der Bildgebungsvorrichtung eine Eingabeoption anzuzeigen. Die Ausgabeeinheit kann als Monitor ausgestaltet sein. Die Ausgabeeinheit kann bevorzugt dazu ausgebildet sein, einem Nutzer der Bildgebungsvorrichtung eine weitere Eingabeoption bereitzustellen und insbesondere mittels eines Monitors anzuzeigen. Die Benutzerschnittstelle kann dazu ausgebildet sein, dem Nutzer eine Eingabe bzw. eine Auswahl eines diagnostisch relevanten Subbereichs zu ermöglichen. Die Benutzerschnittstelle kann weiterhin dazu ausgebildet sein, dem Nutzer ein Anpassen eines (gemeinsamen) Bildgebungsbereichs in Abhängigkeit einer weiteren Eingabeoption zu ermöglichen.

Die Ausgabeeinheit und die Benutzerschnittstelle können bevorzugt in eine Komponente integriert sein. Die Ausgabeeinheit und die Benutzerschnittstelle können aber auch als eigenständige Komponenten ausgestaltet sein. In einer bevorzugten Ausführungsform bilden die Ausgabeeinheit und die Benutzerschnittstelle eine grafische Benutzerschnittstelle, welche dazu ausgebildet ist, dem Nutzer der Bildgebungsvorrichtung eine Eingabeoption zum Erfassen einer Information über einen Subbereich einer Körperregion bereitzustellen und eine Auswahl eines diagnostisch relevanten Subbereichs zu ermöglichen.

Vorzugsweise weist die Steuereinheit eine Signalverbindung mit der Benutzerschnittstelle und/oder der Ausgabeeinheit der Bildgebungsvorrichtung auf. Insbesondere kann die Steuereinheit dazu ausgebildet sein, die Ausgabeeinheit anzusteuern, die Eingabeoption an den Nutzer der Bildgebungsvorrichtung auszugeben. Die Steuereinheit kann weiterhin dazu ausgebildet sein, durch den Nutzer ausgewählte diagnostisch relevante Subbereiche mittels der Benutzerschnittstelle zu erfassen.

Die vorgeschlagene Bildgebungsvorrichtung weist die Vorteile eines vorgeschlagenen Verfahrens gemäß einer der oben beschriebenen Ausführungsformen auf.

Die Komponenten der erfindungsgemäßen Bildgebungsvorrichtung können vorteilhaft aufeinander abgestimmt sein, sodass eine zeiteffiziente und robuste Durchführung eines vorgeschlagenen Verfahrens ermöglicht wird. Insbesondere kann die Bildgebungsvorrichtung dazu ausgebildet sein, einen Verfahrensablauf autark zu koordinieren und durchzuführen. Dem Nutzer der Bildgebungsvorrichtung kann somit ein Auswählen eines Bildgebungsbereichs für eine Bildgebungsuntersuchung durch Auswahl eines oder mehrerer Subbereiche der Körperregion des Patienten ermöglicht werden, ohne dass eine besondere Fachkenntnis des Nutzers oder ein aufwändiges Training erforderlich ist.

Das erfindungsgemäße Computerprogrammprodukt ist direkt in eine Speichereinheit einer Recheneinheit einer erfindungsgemäßen Bildgebungsvorrichtung ladbar. Das Computerprogrammprodukt weist Programmcode-Mittel auf, um ein Verfahren gemäß einer der oben beschriebenen Ausführungsformen durchzuführen, wenn das Computerprogrammprodukt in der Recheneinheit der Bildgebungsvorrichtung ausgeführt wird.

Die Recheneinheit der erfindungsgemäßen Bildgebungsvorrichtung kann bevorzugt von der Steuereinheit umfasst sein oder als eine eigenständige Komponente ausgestaltet sein. Es kann eine Signalverbindung zwischen der Steuereinheit und der Recheneinheit vorgesehen sein.

Durch das vorgeschlagene Computerprogrammprodukt kann das vorgeschlagene Verfahren schnell, identisch wiederholbar und robust ausgeführt werden. Das Computerprogrammprodukt ist so konfiguriert, dass es mittels der Recheneinheit die vorgeschlagenen Verfahrensschritte ausführen kann. Vorzugsweise weist die Recheneinheit erforderliche Voraussetzungen, wie beispielsweise einen Arbeitsspeicher, eine Grafikkarte oder eine Logikeinheit, auf, sodass die jeweiligen Verfahrensschritte effizient ausgeführt werden können. Das Computerprogrammprodukt ist beispielsweise auf einem computerlesbaren Medium gespeichert oder auf einem Netzwerk, einem Server oder einer Cloud hinterlegt, von wo es in einen Prozessor der Recheneinheit geladen werden kann. Die Recheneinheit kann dabei als eine eigenständige Systemkomponente oder als ein Teil der Bildgebungsvorrichtung ausgebildet sein. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Recheneinheit der Bildgebungsvorrichtung ein vorgeschlagenes Verfahren durchführen. Beispiele für elektronisch lesbare Datenträger sind eine DVD, ein Magnetband, ein USB-Stick oder beliebige andere Datenspeicher, auf welchen elektronisch lesbare Steuerinformationen, insbesondere Software, gespeichert ist. Wenn diese Steuerinformationen von dem Datenträger gelesen und an eine Steuereinheit und/oder die Recheneinheit der Bildgebungsvorrichtung übertragen werden, können alle vorgeschlagenen Ausführungsformen des beschriebenen, vorgeschlagenen Verfahrens durchgeführt werden.

Weitere Vorteile und Einzelheiten der vorliegenden Erfindung ergeben sich aus den im Folgenden beschriebenen Ausführungsbeispielen sowie anhand der Zeichnungen. Dabei zeigen:
Fig. 1 eine schematische Repräsentation einer Ausführungsform der erfindungsgemäßen Bildgebungsvorrichtung,
Fig. 2 ein Ablaufdiagramm einer Ausführungsform des erfindungsgemäßen Verfahrens,
Fig. 3 ein Ablaufdiagramm einer Ausführungsform des erfindungsgemäßen Verfahrens mit optionalen Verfahrensschritten,
Fig. 4 eine schematische Repräsentation einer Eingabeoption zum Erfassen einer Information über einen Subbereich in einer Ausführungsform des erfindungsgemäßen Verfahrens mit Erfassung eines diagnostisch relevanten Subbereichs,
Fig. 5 eine schematische Repräsentation einer Eingabeoption zum Erfassen einer Information über einen Subbereich in einer Ausführungsform des erfindungsgemäßen Verfahrens mit Erfassung zweier beabstandeter diagnostisch relevanter Subbereiche,
Fig. 6 eine schematische Repräsentation einer Eingabeoption zum Erfassen einer Information über einen Subbereich in einer Ausführungsform des erfindungsgemäßen Verfahrens mit Erfassung zweier benachbarter diagnostisch relevanter Subbereiche mit gemeinsamem Bildgebungsbereich,
Fig. 7 eine schematische Repräsentation einer Eingabeoption zum Erfassen einer Information über einen Subbereich in einer Ausführungsform des erfindungsgemäßen Verfahrens mit Erfassung zweier benachbarter diagnostisch relevanter Subbereiche mit jeweiligen Bildgebungsbereichen,
Fig. 8 eine schematische textbasierte Repräsentation einer Eingabeoption zum Erfassen einer Information über einen Subbereich in einer Ausführungsform des erfindungsgemäßen Verfahrens mit einer weiteren Eingabeoption.

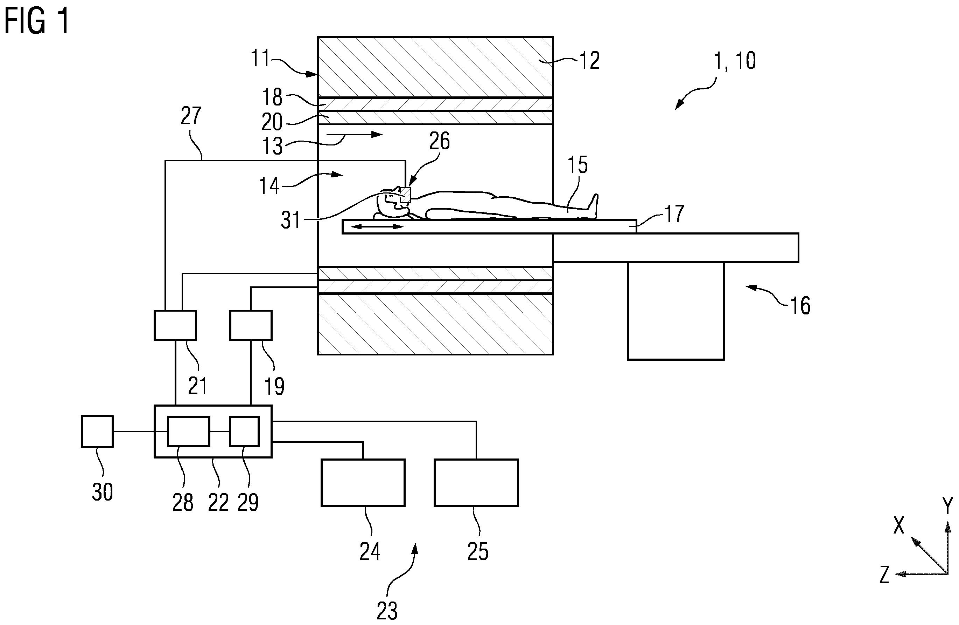

In Fig. 1 ist eine Ausführungsform der erfindungsgemäßen Bildgebungsvorrichtung 1 dargestellt. Die Bildgebungsvorrichtung 1 ist vorliegend als ein Magnetresonanzvorrichtung 10 ausgestaltet. Das Magnetresonanzvorrichtung 10 umfasst eine Magneteinheit 11, welche z. B. einen Permanentmagneten, einen Elektromagneten oder einen supraleitenden Hauptmagneten 12 zur Erzeugung eines starken und insbesondere homogenen Hauptmagnetfelds 13 (B0-Magnetfeld) aufweist. Zudem umfasst das Magnetresonanzvorrichtung 10 einen Patientenaufnahmebereich 14 zu einer Aufnahme eines Patienten 15. Der Patientenaufnahmebereich 14 ist im vorliegenden Ausführungsbeispiel zylinderförmig ausgebildet und in einer Umfangsrichtung von der Magneteinheit 11 umgeben. Grundsätzlich sind jedoch auch von diesem Beispiel abweichende Ausbildungen des Patientenaufnahmebereichs 14 vorstellbar.

Der Patient 15 kann mittels einer Patientenlagerungsvorrichtung 16 der Magnetresonanzvorrichtung 10 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf.

Das Magnetresonanzvorrichtung 10 weist weiterhin eine Gradientenspule 18 zum Erzeugen von magnetischen Gradientenfeldern auf, welche für eine Ortskodierung während einer Magnetresonanzmessung verwendet wird. Die Gradientenspule 18 wird mittels einer Gradientensteuereinheit 19 der Magnetresonanzvorrichtung 10 angesteuert.

Das Magnetresonanzvorrichtung 10 kann weiterhin eine Hochfrequenzantenne umfassen, welche im vorliegenden Ausführungsbeispiel als fest in das Magnetresonanzvorrichtung 10 integrierte Körperspule 20 ausgebildet ist. Die Körperspule 20 ist zu einer Anregung von Kernspins ausgelegt, welche sich in dem von dem Hauptmagneten 12 erzeugten Hauptmagnetfeld 13 befinden. Die Körperspule 20 wird von einer Hochfrequenzeinheit 21 der Magnetresonanzvorrichtung 10 angesteuert und strahlt hochfrequente Anregungspulse in eine Bildaufnahmeregion ein, die im Wesentlichen von dem Patientenaufnahmebereich 14 der Magnetresonanzvorrichtung 10 gebildet ist. Die Körperspule 20 kann als eine Empfangseinheit der Magnetresonanzvorrichtung 10 ausgestaltet sein, welche dazu ausgebildet ist, Magnetresonanzsignale aus dem Patientenaufnahmebereich 14 zu empfangen.

Zu einer Steuerung des Hauptmagneten 12, der Gradientensteuereinheit 19 und der Hochfrequenzeinheit 21 weist die Magnetresonanzvorrichtung 10 eine Steuereinheit 22 auf. Die Steuereinheit 22 ist dazu ausgebildet, eine Durchführung einer Bildgebungssequenz der Bildgebungsuntersuchung, wie z. B. einer GRE (gradient echo) Sequenz, einer TSE (turbo spin echo) Sequenz oder einer UTE (ultra-short echo time) Sequenz, zu steuern. Zudem umfasst die Steuereinheit 22 eine Recheneinheit 28 zu einer Auswertung von Magnetresonanzsignalen, die während einer Magnetresonanzuntersuchung erfasst werden.

Des Weiteren umfasst das Magnetresonanzvorrichtung 10 eine Benutzerschnittstelle 23, welche eine Signalverbindung mit der Steuereinheit 22 aufweist. Steuerinformationen, wie z. B. Bildgebungsparameter der Magnetresonanzuntersuchung, aber auch rekonstruierte Bilddaten einer Körperregion 31 des Patienten 15, sowie eine Hilfestellung zur Anpassung eines Bildgebungsparameters, können auf einer Ausgabeeinheit 24 der Benutzerschnittstelle 23 für einen Nutzer angezeigt werden. Die Ausgabeeinheit 24 kann hierfür beispielsweise einen oder mehrere Monitore umfassen. Die Ausgabeeinheit 24 kann insbesondere dazu ausgelegt sein, eine grafische Benutzeroberfläche mit schematischen Bilddaten der Körperregion 31 des Patienten und eine Eingabeoption zur Auswahl eines diagnostisch relevanten Subbereichs bereitzustellen. Vorzugsweise weist die Benutzerschnittstelle 23 eine Eingabeeinheit 25 auf, welche dem Nutzer insbesondere eine Auswahl eines diagnostisch relevanten Bereichs und/oder ein Anpassen von Bildgebungsparametern ermöglicht. Die Eingabeeinheit 25 kann ferner dazu ausgebildet sein, dem Nutzer eine Anpassung einer Dimension, einer Ausrichtung und/oder einer Position eines grafischen Objekts, welches einen gemeinsamen Bildgebungsbereich mehrerer Subbereiche einer Körperregion repräsentiert, in Abhängigkeit von erfassten Subbereichen (32a,32b) der Körperregion 31 des Patienten 15 und einer weiteren Eingabeoption zur Anpassung des gemeinsamen Bildgebungsbereichs zu ermöglichen.

Die Recheneinheit 28 ist in dem vorliegenden Beispiel mittels einer Signalverbindung mit einer Speichereinheit 29 der Magnetresonanzvorrichtung 10 verbunden. Optional kann die Recheneinheit 28 auch mittels einer Signalverbindung mit einer Cloud 30 verbunden sein. Die Recheneinheit 28 kann dazu ausgebildet sein, Daten wie Patienteninformationen, Bilddaten, Localizer-Bilddaten, Magnetresonanzbilder, Röntgenbilder oder dergleichen, auf der Speichereinheit 29 und/oder der Cloud 30 zu speichern und/oder entsprechende Daten von der Speichereinheit 29 und/oder der Cloud 30 mittels einer geeigneten Schnittstelle (nicht dargestellt) abzurufen. Es ist vorstellbar, dass die Recheneinheit 28 dazu ausgebildet ist, eine Patienteninformation des Patienten 15 von der Cloud 30 und/oder der Speichereinheit 29 zu beziehen. Es ist ebenso vorstellbar, dass die Recheneinheit 28 dazu ausgebildet ist, eine Information über die Körperregion 31 des Patienten 15 in Abhängigkeit einer Patienteninformation, insbesondere eines Namens und/oder einer anderen Identifikation, von der Cloud 30 und/oder der Speichereinheit 29 zu beziehen.

In einer bevorzugten Ausführungsform weist die Magnetresonanzvorrichtung 10 eine Dentalspule 26 auf, welche in einer anwendungsgemäßen Position an der Kieferregion 31 und/oder in einer Mundhöhle des Patienten 15 positioniert ist. Die Dentalspule 26 kann ein Antennenelement (nicht gezeigt) aufweisen, welche dazu ausgebildet ist, Magnetresonanzsignale der Kieferregion und/oder der Zahnregion des Patienten 15 zu erfassen und an die Recheneinheit 28 und/oder die Steuereinheit 22 übermitteln.

Die Dentalspule 26 weist vorliegend eine elektrische Anschlussleitung 27 auf, welche eine Signalverbindung mit der Hochfrequenzeinheit 21 bereitstellt. In einer bevorzugten Ausführungsform ist die Dentalspule 26 dazu ausgebildet, Kernspins in der Kieferregion 31 des Patienten 15 anzuregen. Die Dentalspule 26 kann hierfür von der Hochfrequenzeinheit 21 angesteuert werden. In einem Beispiel ist die Dentalspule 26 als eine Maske ausgeführt, welche in einer anwendungsgemäßen Position an einer Hautoberfläche der Kieferregion 31 des Patienten 15 positioniert ist. Es ist jedoch ebenso vorstellbar, dass die Dentalspule 26 mechanisch mit einem Beißelement verbunden ist, welches in einer anwendungsgemäßen Position an einem Zahnbogen, insbesondere entlang einer Okklusionsebene, des Patienten 15 positioniert ist.

Die vorgeschlagene Magnetresonanzvorrichtung 10 kann selbstverständlich weitere Komponenten umfassen, welche Magnetresonanzvorrichtungen üblicherweise aufweisen. Es ist ebenso vorstellbar, dass die Magnetresonanzvorrichtung 10 statt des zylinderförmigen Aufbaus einen C-förmigen, einen dreieckigen oder einen asymmetrischen Aufbau der Magnetfelderzeugenden Komponenten aufweist. Das Magnetresonanzvorrichtung 10 kann weiterhin dazu ausgebildet ein, eine Magnetresonanzuntersuchung eines stehenden oder sitzenden Patienten 15 durchzuführen.

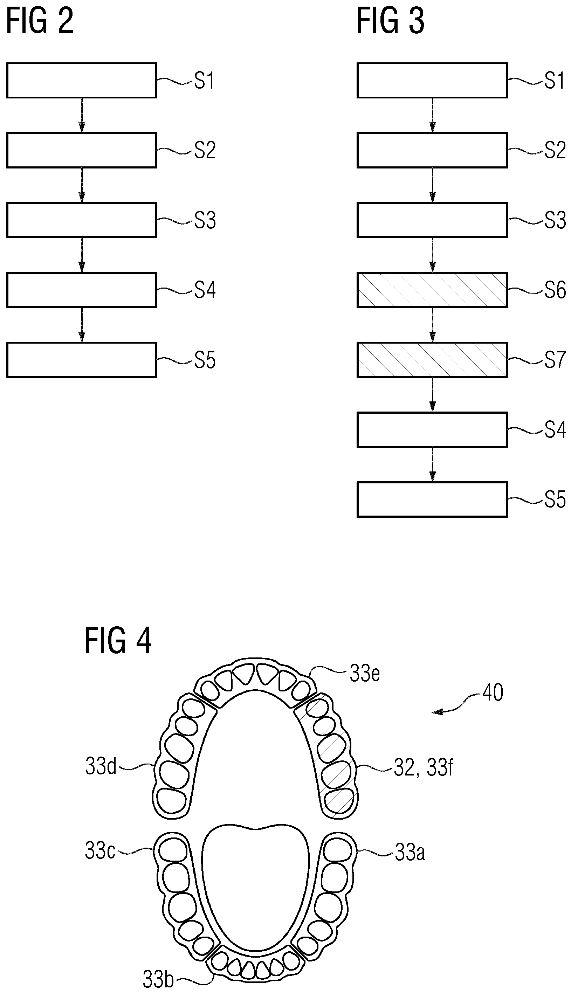

In Fig. 2 ist ein Ablaufdiagramm einer Ausführungsform des vorgeschlagenen Verfahrens zur Erfassung von Bilddaten eines diagnostisch relevanten Subbereichs einer Körperregion 31 eines Patienten dargestellt. In Fig. 3 ist ein Ablaufdiagramm einer weiteren Ausführungsform des vorgeschlagenen Verfahrens zur Erfassung von Bilddaten eines diagnostisch relevanten Subbereichs einer Körperregion eines Patienten 15 mit optionalen Verfahrensschritten dargestellt. Der Ablauf der beispielhaften Ausführungsformen des Verfahrens wird im Folgenden anhand einer Magnetresonanzvorrichtung 10 erläutert. Selbstverständlich kann das erfindungsgemäße Verfahren auch mittels einer anderen Bildgebungsvorrichtung gemäß einer oben beschriebenen Ausführungsform ausgeführt werden.

In dem Schritt S1 werden Informationen über die Körperregion 31 des Patienten erfasst. Es ist vorstellbar, dass die Information über die Körperregion des Patienten 15 von einem Nutzer der Magnetresonanzvorrichtung 10 mittels der Benutzerschnittstelle 23 eingegeben werden. Die Benutzerschnittstelle 23 kann hierfür eine beliebige Eingabeeinheit 25 umfassen, welche eine Interaktion des Nutzers mit einer grafischen Benutzerschnittstelle ermöglicht. Beispielsweise weist die Eingabeeinheit 25 eine Tastatur, eine Maus und/oder einen Touchscreen auf. Die von dem Nutzer eingegebene Information über die Körperregion des Patienten kann anschließend mittels der Steuereinheit 22 und/oder der Recheneinheit 28 der Magnetresonanzvorrichtung 10 erfasst und verarbeitet werden. Alternativ kann die Information über die Körperregion des Patienten vorbelegt sein, wenn die Magnetresonanzvorrichtung 10 lediglich zu der Aufnahme einer vorbestimmten Körperregion eingesetzt wird. Die Information über die Körperregion des Patienten kann ebenso in Abhängigkeit einer Patienteninformation von einem radiologischen Informationssystem, einem Krankenhausinformationssystem, einer Netzwerk-Speichereinheit, einer lokalen Speichereinheit 29 und/oder einer Cloud 30 erfasst oder abgefragt werden.

In einem Schritt S2 wird eine Eingabeoption bereitgestellt, durch die eine Information über einen Subbereich der Körperregion erfasst werden kann. Das Bereitstellen der Eingabeoption erfolgt hierbei in Abhängigkeit der Information über die Körperregion. Die Körperregion umfasst mehrere Subbereiche, die durch die Eingabeoption auswählbar sind. Vorzugsweise wird die Eingabeoption zur Erfassung einer Information über den Subbereich, insbesondere zu einer Auswahl eines diagnostisch relevanten Subbereichs, dem Nutzer der Magnetresonanzvorrichtung 10 mittels der Ausgabeeinheit 25 bereitgestellt. Alternativ kann ein Parameter eines Subbereichs oder auch andere Informationen über einen Subbereich erfasst werden. Die Erfassung einer Anpassung eines Parameters eines Subbereichs und/oder einer anderen Information kann jedoch insbesondere über eine weitere Eingabeoption (siehe Schritte S6 und S7) erfolgen. Das Bereitstellen der Eingabeoption kann insbesondere ein Anzeigen einer schematischen grafischen Abbildung der Körperregion auf einem Monitor der Benutzerschnittstelle 23 umfassen.

Vorzugsweise stellen die grafische (oder textbasierte) Repräsentation der Körperregion des Patienten und die Eingabeoption zur Erfassung der Information über den Subbereich einen Teil einer Ausgabe 40 (siehe Figuren 3 bis 7) der Benutzerschnittstelle 23 dar, welche dem Nutzer ein Bestimmen eines Bildgebungsprotokolls einer Magnetresonanzuntersuchung, insbesondere durch Bestimmung eines diagnostisch relevanten Subbereichs und/oder beispielsweise eines Parameters eines gemeinsamen Bildgebungsbereichs mehrerer Subbereiche, für eine Bildgebungsuntersuchung ermöglicht.

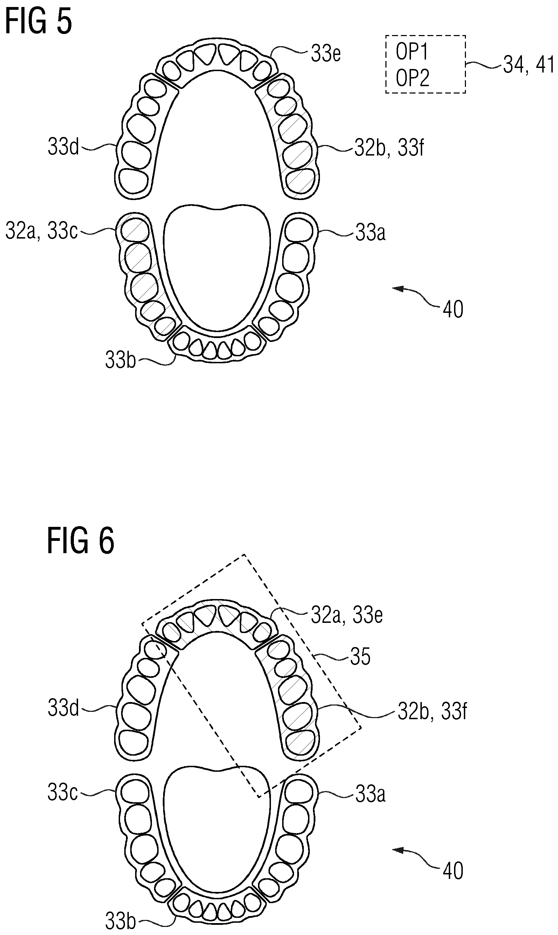

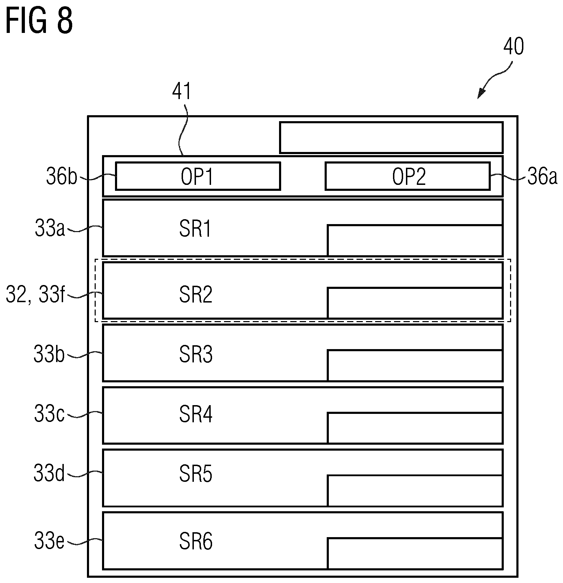

Die Eingabeoption zum Erfassen der Information über den Subbereich kann beispielsweise eine textbasierte Eingabemaske und/oder ein textbasiertes Eingabefenster (siehe Figur 8) umfassen. Bevorzugt umfasst die Eingabeoption, die ebenfalls eine Anpassung eines Parameters eines (gemeinsamen) Bildgebungsbereichs ermöglichen kann (siehe Figur 6), eine grafische Repräsentation der Körperregion oder ein grafisches Objekt (siehe Figuren 4 bis 6), welches dem Nutzer ein Anwählen von Subbereichen ermöglichen kann.

In einem weiteren Schritt S3 wird ein diagnostisch relevanter Subbereich einer Körperregion erfasst. Vorzugsweise wird mindestens ein durch den Nutzer mittels der Benutzerschnittstelle 23 angewählter Subbereich der Körperregion von der Steuereinheit 22 und/oder der Recheneinheit 28 erfasst. Das Erfassen einer Information über einen Subbereich, insbesondere eines diagnostisch relevanten Subbereichs, kann ein Erfassen einer Eingabe mittels einer Maus, einer Tastatur und/oder einer Touch-Eingabe eines Touchscreens umfassen. Das Erfassen des diagnostisch relevanten Subbereichs kann insbesondere ein Speichern des diagnostisch relevanten Subbereichs auf der Speichereinheit 29, einem Cloud-Speicher 30, einem Netzwerk-Speicher oder dergleichen umfassen.

In einem optionalen Schritt S6 kann das Bereitstellen einer weiteren Eingabeoption zum Erfassen einer weiteren Information über einen diagnostisch relevanten Subbereich der Körperregion erfolgen. Die weitere Information über den diagnostisch relevanten Subbereich kann insbesondere einen Parameter eines Subbereichs bzw. eines Bildgebungsbereich und/oder beispielsweise die Auswahl eines Teilbereichs des Subbereichs einer Körperregion des Patienten umfassen. Beispielsweise können die Steuereinheit 22 und/oder die Recheneinheit 28 dazu ausgebildet sein, in Abhängigkeit der durch den Nutzer ausgewählten Subbereiche einen vorläufigen Bildgebungsbereichs und/oder ein grafisches Objekt, welches einen gemeinsamen Bildgebungsbereich mehrerer Subbereiche repräsentiert, zu ermitteln. Beispielsweise kann der Nutzer eine Dimension, eine Ausrichtung und/oder eine Position der Repräsentation des (gemeinsamen) Bildgebungsbereichs anpassen, um den Parameter des (gemeinsamen) Bildgebungsbereichs oder eines Bildgebungsprotokolls eines Subbereichs anzupassen. Vorzugsweise sind die Steuereinheit 22 und/oder die Recheneinheit 28 der Magnetresonanzvorrichtung 10 dazu ausgebildet, die Anpassung des Parameters anhand der vom Nutzer geänderten Repräsentation des (gemeinsamen) Bildgebungsbereichs abzuleiten oder zu ermitteln.

Vorzugsweise wird der vorläufige Bildgebungsbereich automatisch mittels der Steuereinheit 22 und/oder der Recheneinheit 28 des Magnetresonanzgeräts 10 in Abhängigkeit des erfassten diagnostisch relevanten Subbereichs der Körperregion 31 des Patienten 15 ermittelt. Die Steuereinheit 22 und/oder die Recheneinheit 28 kann einen Algorithmus umfassen, welcher dazu ausgebildet ist, für einen oder mehrere diagnostisch relevante Subbereiche der Körperregion 31 des Patienten 15 einen vorläufigen und/oder gemeinsamen Bildgebungsbereich zu ermitteln und/oder bereitzustellen.

In einem optionalen Schritt S7 kann eine Eingabe in Abhängigkeit der weiteren Eingabeoption zum Erfassen der weiteren Information über den diagnostisch relevanten Subbereich erfasst werden. Vorzugsweise wird mindestens ein durch den Nutzer mittels der Benutzerschnittstelle 23 angepasster Bildgebungsparameter des Bildgebungsprotokolls von der Steuereinheit 22 und/oder der Recheneinheit 28 erfasst. Alternativ kann die weitere Information über den diagnostisch relevanten Subbereich eine Anzahl von Bildgebungsuntersuchungen, eine Reihenfolge von Bildgebungsuntersuchungen oder einen erfassten Teilbereich eines Subbereichs einer Körperregion umfassen. Das Erfassen einer weiteren Information über den diagnostisch relevanten Subbereich kann ein Erfassen einer Eingabe mittels einer Maus, einer Tastatur und/oder einer Touch-Eingabe eines Touchscreens umfassen. Das Erfassen der weiteren Information kann insbesondere ein Speichern der weiteren Information auf der Speichereinheit 29, einem Cloud-Speicher 30, einem Netzwerk-Speicher oder dergleichen umfassen.

In einem Schritt S4 wird ein Bildgebungsprotokoll zur Erfassung von Bilddaten des diagnostisch relevanten Subbereichs der Körperregion des Patienten ermittelt. Die Ermittlung des Bildgebungsprotokolls erfolgt für den erfassten diagnostisch relevanten Subbereich. Die Ermittlung des Bildgebungsprotokolls für eine Bildgebungsuntersuchung kann insbesondere automatisch auf Basis des erfassten diagnostisch relevanten Subbereichs erfolgen. Vorzugsweise wird das Ermitteln des Bildgebungsprotokolls für die Bildgebungsuntersuchung automatisch mittels der Steuereinheit 22 und/oder der Recheneinheit 28 durchgeführt. Die Recheneinheit 28 kann einen Algorithmus umfassen, welcher dazu ausgebildet ist, das Bildgebungsprotokoll für die Bildgebungsuntersuchung in Abhängigkeit der erfassten diagnostisch relevanten Subbereiche zu ermitteln. Beispielsweise kann der Algorithmus dazu ausgebildet sein, eine durch den Nutzer vorgenommene Veränderung einer Dimension, einer Position und/oder einer Ausrichtung des vorläufigen (oder gemeinsamen) Bildgebungsbereichs zu erfassen und in ein Bildgebungsprotokoll für die Bildgebungsuntersuchung zu übersetzen.

Die Steuereinheit 22 und/oder die Recheneinheit 28 kann jedoch ebenso dazu ausgebildet sein, das Bildgebungsprotokoll und/oder Bildgebungsparameter anhand von numerischen Daten und/oder einer textbasierten Eingabe des Nutzers zu ermitteln. Der Nutzer kann hierfür eine entsprechende Eingabe mittels der benutzerschnittstelle 23; 25 vornehmen.

In einem Schritt S5 wird das ermittelte Bildgebungsprotokoll zur Erfassung von Bilddaten des diagnostisch relevanten Subbereichs 32 der Körperregion 31 mittels der Bildgebungsvorrichtung 1 bereitgestellt. Die Benutzerschnittstelle 23; 25 und/oder die Recheneinheit 28 können dazu ausgebildet sein, das Bildgebungsprotokoll an die Steuereinheit 22 zu übermitteln. Die Steuereinheit 22 kann insbesondere die Durchführung einer Bildgebungsuntersuchung mittels der Magnetresonanzvorrichtung 10 anhand des ermittelten und/oder bereitgestellten Bildgebungsprotokolls steuern.

Zur Durchführung der Bildgebungsuntersuchung kann der Patient 15, wie in Fig. 1 gezeigt, anwendungsgemäß in dem Patientenaufnahmebereich 14 der Magnetresonanzvorrichtung 10 positioniert sein. Die Bildgebungsuntersuchung kann beispielsweise eine Magnetresonanzuntersuchung eines Subbereichs einer Zahnregion, beispielsweise der Schneidezahnregion, des Patienten 15 sein.

In Fig. 4 ist eine schematische Repräsentation einer Eingabeoption 40 zum Erfassen einer Information über einen Subbereich 33a-33f in einer Ausführungsform des vorgeschlagenen Verfahrens mit Erfassung eines diagnostisch relevanten Subbereichs 32, 33f dargestellt. Die beispielhafte Darstellung in Fig. 4 kann insbesondere eine Ausgabe 40 der Ausgabeeinheit 25 und/oder der Benutzerschnittstelle 23 für einen Nutzer der Bildgebungsvorrichtung 1 repräsentieren.

Das Bereitstellen der Eingabeoption 40 zum Erfassen einer Information über einen diagnostisch relevanten Subbereichs 32, 33f gemäß des Schritts S2 umfasst vorliegend ein Bereitstellen einer grafischen Repräsentation der Körperregion. Das Erfassen S3 des diagnostisch relevanten Subbereichs 32 umfasst entsprechend eine grafische Auswahl des Subbereichs 33a-33f der Körperregion des Patienten anhand der grafischen Repräsentation der Körperregion durch den Nutzer. Der diagnostisch relevante Subbereichs 32, 33f kann dem Nutzer in Abhängigkeit der Information über die Körperregion 31, einer Patienteninformation, Bilddaten aus vorangegangenen Untersuchungen und/oder einer Untersuchungsanweisung automatisch vorgeschlagen werden und ein idealer Bildgebungsbereich für den spezifischen (diagnostisch relevanten) Subbereich der Körperregion vorkonfiguriert werden. Insbesondere die grafische Auswahl eines Subbereichs 33a-33f der Körperregion und/oder ein vorgeschlagener Subbereich der Körperregion können in der Eingabeoption grafisch oder farbig herausgestellt sein bzw. werden. Beispielsweise können die Steuereinheit 22 und/oder die Recheneinheit 28 dazu ausgebildet sein, den diagnostisch relevanten Subbereich 32, 33f in Abhängigkeit der Information über die Körperregion 31 des Patienten 15, einer Patienteninformation, Bilddaten aus vorangegangenen Untersuchungen und/oder einer Untersuchungsanweisung auszuwählen oder zu ermitteln. Die Eingabeoption 40 zum Erfassen der Information über den diagnostisch relevanten Subbereich 32, 33f kann anderes als dargestellt insbesondere eine grafische Anleitung zur Auswahl eines oder mehrerer Subbereiche 32, 33f der Körperregion umfassen.

In Fig. 5 ist eine schematische Repräsentation einer Eingabeoption 40 zum Erfassen einer Information über einen Subbereich 33a-33f in einer weiteren Ausführungsform des vorgeschlagenen Verfahrens mit Erfassung eines diagnostisch relevanten Subbereichs 32, 33f dargestellt. Die beispielhafte Darstellung in Fig. 4 kann insbesondere eine Ausgabe der Eingabeoption 40 mittels der Ausgabeeinheit 25 und/oder der Benutzerschnittstelle 23 an einen Nutzer der Bildgebungsvorrichtung 1 repräsentieren.

Im Unterschied zu der in Fig. 4 gezeigten Ausführungsform, umfasst die gezeigte Ausführungsform eine weitere Eingabeoption 41 die in einem Fenster 34 angezeigt werden kann. Zudem verdeutlicht die in Fig. 5 gezeigte Ausführungsform den Prozess einer Auswahl von mehr als einem diagnostisch relevanten Subbereich 32a, 33f, 32b, 33c mittels der Eingabeoption 40. Die durch einen Nutzer bestimmten diagnostisch relevanten Subbereiche 32a, 33f, 32b, 33c sind in diesem Beispiel beabstandet voneinander angeordnet, sodass bevorzugt jeweils ein Bildgebungsprotokoll gemäß eines Schrittes S3 einer Ausführungsform des oben vorgeschlagenen Verfahrens für die bestimmten diagnostisch relevanten Subbereiche 32a, 33f, 32b, 33c erfolgt.

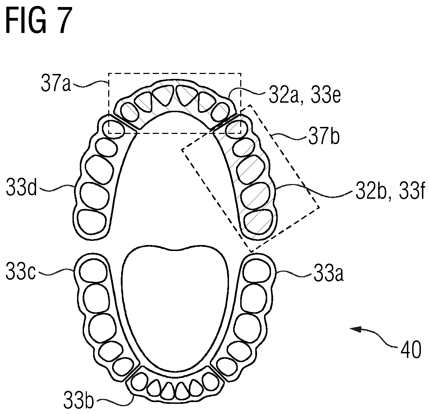

Die Eingabeoption 41 kann zur Anpassung eines Parameters des Bildgebungsprotokolls für einen der erfassen diagnostisch relevanten Subbereiche 32a, 33f, 32b, 33c ein Textfeld 34 zur Eingabe von Parameterwerten umfassen. Beispielsweise können der Parameter OP1 eine Dimension und der Parameter OP2 eine Ausrichtung eines Bildgebungsbereichs eines der diagnostisch relevanten Subbereiche 32a, 33f, 32b, 33c definieren. Daneben ist auch eine Eingabe weiterer Parameter, welche für die jeweilige Bildgebungsvorrichtung 1 relevant sind, möglich. Es ist weiterhin vorstellbar, dass die Eingabeoption 41 zur Anpassung eines Parameters wie in Fig. 6 gezeigt, ein grafisches Objekt umfasst, welches dem Nutzer eine grafische Anpassung eines (gemeinsamen) Bildgebungsbereichs 35 ermöglicht. Vorzugsweise werden die Parameter OP1, OP2 in Abhängigkeit einer Manipulation des grafischen Objekts durch den Nutzer aktualisiert. Alternativ ist denkbar, dass die weitere Eingabeoption 41 eine Auswahl aus beispielsweise zwei Optionen OP1, OP2 für einen Nutzer bereitstellt. Insbesondere aufgrund der Auswahl von diagnostisch relevanten Subbereichen 32a, 33f, 32b, 33c mittels der Eingabeoption 40 kann ein weiterer Nutzerinput notwendig werden. Beispielweise kann der Nutzer wie dargestellt beabstandete diagnostisch relevanten Subbereiche 32a, 33f, 32b, 33c bestimmen. In diesem Fall ermöglicht die weitere Eingabeoption 41 dem Nutzer in einem aufgrund der Auswahl interaktiv anzeigten Fenster 34 eine Festlegung, ob eine gemeinsame Bildgebung der diagnostisch relevanten Subbereiche 32a, 33f, 32b, 33c mittels eines gemeinsamen Bildgebungsbereichs entsprechend einer ersten Option OP1 oder die Bereitstellung zweier Bildgebungsprotokolle für zwei Bildgebungsuntersuchungen für jeden der erfassten diagnostisch relevanten Subbereiche 32a, 33f, 32b, 33c entsprechend einer zweiten Option OP2 erfolgen soll. Die zweite Option OP2 ist in Fig. 7 verdeutlicht.

In Fig. 6 ist eine schematische Repräsentation einer Eingabeoption 40 zum Erfassen einer Information über einen Subbereich 33a-33f in einer weiteren Ausführungsform des vorgeschlagenen Verfahrens mit Erfassung eines diagnostisch relevanten Subbereichs 32, 33f dargestellt. Die beispielhafte Darstellung in Fig. 4 kann insbesondere eine Ausgabe 40 der Ausgabeeinheit 25 und/oder der Benutzerschnittstelle 23 für einen Nutzer der Bildgebungsvorrichtung 1 repräsentieren.

Im Unterschied zu den in Fig. 4 und Fig. 5 gezeigten Ausführungsformen, umfasst die Ausführungsform der Fig. 6 ein Erfassen einer weiterer Eingabeoption 41, die durch die grafische Repräsentation eines gemeinsamen Bildgebungsbereich 35 ausgebildet ist. Beispielsweise kann diese Eingabeoption ein Auswahlfenster (nicht dargestellt) umfassen, welches die Auswahl einer Mehrzahl von benachbarten Subbereichen 32a, 33e, 32b, 33f erlaubt. Auch die in Fig. 6 gezeigte Ausführungsform verdeutlicht die Auswahl von mehr als einem diagnostisch relevanten Subbereich 32a, 33e, 32b, 33f mittels der Eingabeoption 40. Die durch einen Nutzer bestimmten diagnostisch relevanten Subbereiche 32a, 33e, 32b, 33f sind in diesem Beispiel im Unterschied zu der Abbildung in Fig. 5 benachbart zueinander angeordnet, sodass bevorzugt die Ermittlung eines Bildgebungsprotokolls in Abhängigkeit eines gemeinsamen Bildgebungsbereichs 35 gemäß eines Schrittes S3 einer Ausführungsform des oben vorgeschlagenen Verfahrens erfolgt.

Die Körperregion kann wie in Fig. 6 durch zwei schematische Schnittansichten der Körperregion des Patienten 15 dargestellt sein, kann alternativ aber auch durch eine dreidimensionale Modellansicht abgebildet sein. Die Eingabeoptionen 41 ist vorliegend als grafisches Objekt ausgestaltet, welche durch den Nutzer manipulierbar ist. Vorzugsweise werden eine Dimension, eine Ausrichtung und/oder eine Position des grafischen Objekts der Eingabeoptionen 41 aktualisiert, wenn der Nutzer eine Anpassung des gemeinsamen Bildgebungsbereichs 35 vorgenommen hat.

Eine Bereitstellung einer Übersichtsansicht, vergleichbar mit der Abbildung der Körperregion 31 in den Figuren 4 bis 6, ist beispielsweise auch für den Schritt S1 als Eingabeoption für den Nutzer denkbar, um unterschiedlichen Körperreiche eines Patienten 15 abzubilden und die Information über die Körperregion 31 des Patienten 15 zu erfassen.

In einer Ausführungsform des vorgeschlagenen Verfahrens erfolgt das Bereitstellen einer Hilfestellung beispielweise zur Anpassung des gemeinsamen Bildgebungsbereichs in Abhängigkeit der erfassten diagnostisch relevanten Subbereiche der Körperregion 31 durch eine Ausgabe einer textbasierten Anleitung. In dem in Fig. 6 gezeigten Beispiel kann die Hilfestellung beispielweise die textbasierte Anleitung in Form von zwei nicht dargestellten Anweisungen umfassen, welche Instruktionen zum Ablauf der Anpassung des gemeinsamen Bildgebungsbereichs 35 umfassen. Beispielsweise weist eine erste Anweisung den Nutzer dazu an, den gemeinsamen Bildgebungsbereich 35 in einer für eine Bildgebungsuntersuchung vorteilhaften Ausrichtung, insbesondere einem bestimmten Winkel, zu positionieren. Eine zweite Anweisung kann den Nutzer beispielweise dazu anweisen, die Lage und/oder Dimensionierung des gemeinsamen Bildgebungsbereich 35 so auszurichten, sodass alle ausgewählten (markierten) diagnostisch relevanten Subbereiche von dem gemeinsamen Bildgebungsbereich 35 umfasst sind.

In Fig. 7 ist eine schematische Repräsentation einer Eingabeoption 40 zum Erfassen einer Information über einen Subbereich 33a-33f sowie ein Erfassen eines diagnostisch relevanten Subbereichs 32 in Abhängigkeit der Eingabeoption 40 gemäß einer Ausführungsform des vorgeschlagenen Verfahrens dargestellt. In dem vorliegenden Beispiel ist die Kennzeichnung oder Auswahl von zwei Subbereichen 33e, 33f als diagnostisch relevante Subbereiche 32a, 32b durch einen Nutzer mittels der Eingabeoption 40 verdeutlicht. Für jeden der diagnostisch relevanten Subbereiche 32a und 32b kann gemäß einer oben beschriebenen Ausführungsform eine automatische Anpassung der Bildgebungsbereiche 37a und 37b an die Anatomie des Subbereichs erfolgen.

In den Figuren 3 bis 7 ist die Körperregion 31 des Patienten 15 eine Kieferregion und/oder eine Zahnregion des Patienten 15. Das vorgeschlagenen Verfahrens kann jedoch auch eine beliebige andere Körperregion, insbesondere eine Wirbelsäule, des Patienten 15 betreffen. Analog zu den oben beschriebenen Ausführungsformen kann der Bereich der Wirbelsäule des Patienten 15 durch eine grafische Repräsentation einer Eingabeoption 40 abgebildet sein und dem Nutzer eine Auswahl eines Subbereichs der Wirbelsäule, also beispielsweise den unteren Wirbelsäulenbereich mit drei umfassten Wirbeln, ermöglichen.

Die in den Figuren 4, 5, 6 und 7 dargestellte Eingabeoption 40, bzw. die grafische Repräsentation der Körperregion des Patienten 15 vorliegend eine zweidimensionale Repräsentation, insbesondere eine Schnittansicht, der Zahnregion des Patienten 15. Die Bezugsebenen der Schnittansicht der Körperregion des Patienten 15 sind in dem gezeigten Beispiel parallel zu der Transversalebene des Patienten 15 ausgerichtet. Die Körperregion wird also mit anderen Worten aufgeklappt dargestellt.

Eine weitere Ausgestaltung der Eingabeoption 40 als textbasierte Auswahl des diagnostisch relevanten Subbereichs 32, 33f der Körperregion 31 ist in Fig. 8 dargestellt. Die Eingabeoption umfasst in dieser Ausführungsform eine Listendarstellung der Subbereiche 33a-33f der Körperregion. Eine Auswahl des diagnostisch relevanten Subbereichs 32, 33f ist einem Nutzer beispielweise durch das Anwählen eines oder mehrerer Listenelemente SR1 bis SR6 möglich. Ein ausgewähltes Listenelement kann dem Nutzer vorzugsweise durch eine Umrahmung oder eine farbige Markierung hervorgehoben werden. Auch die weitere Eingabeoption 41 kann als eine textbasierte Auswahl ausgestaltet sein. Beispielweise können zwei Auswahloptionen OP1, OP2 als Textfelder 36a, 36b durch einen Nutzer auswählbar sein.

Selbstverständlich kann das erfindungsgemäße Verfahren zur Bestimmung eines Bildgebungsprotokolls zur Erfassung von Bilddaten beliebiger anderer Körperregionen verwendet werden. Insbesondere kann das erfindungsgemäße Verfahren zur Bestimmung eines Bildgebungsprotokolls zur Erfassung von Bilddaten von Körperregionen mit einer Vielzahl von unterschiedlich ausgerichteten Abschnitten oder Subregionen, wie z. B. einer Hand, einem Fuß, einer Zahnregion, aber auch anderen anatomischen Strukturen und/oder Organen, verwendet werden.

Die hier beschriebenen Ausführungsformen des erfindungsgemäßen Verfahrens und der erfindungsgemäßen Bildgebungsvorrichtung sind als beispielhaft zu verstehen. Wenn nicht im Detail anders erläutert, sind einzelne Ausführungsformen grundsätzlich um Merkmale anderer Ausführungsformen erweiterbar. Insbesondere ist die Reihenfolge der Verfahrensschritte des erfindungsgemäßen Verfahrens als beispielhaft zu verstehen. Die einzelnen Schritte können auch in einer anderen Reihenfolge durchgeführt werden oder sich zeitlich teilweise oder vollständig überschneiden. Beispielsweise können die Schritte des Erfassens der Information über die Körperregion und des Durchführens der ersten Bildgebungsuntersuchung in einer beliebigen Reihenfolge nacheinander oder zumindest teilweise überlappend ablaufen. Gleichermaßen kann das Bereitstellen der Bilddaten und der Eingabeoption zur Anpassung des Parameters des Bildgebungsbereichs sowie das Bereitstellen der Hilfestellung zur Anpassung des Bildgebungsbereichs in Abhängigkeit der Information über die Körperregion in einer beliebigen Reihenfolge nacheinander oder zumindest teilweise überlappend durchgeführt werden.

Unabhängig vom grammatikalischen Geschlecht eines bestimmten Begriffes sind Personen mit männlicher, weiblicher oder anderer Geschlechteridentität mit umfasst.

## Patentansprüche

1. Computer-implementiertes Verfahren zur Bestimmung eines Bildgebungsprotokolls zur Erfassung von Bilddaten eines diagnostisch relevanten Subbereichs (32) einer Körperregion (31) eines Patienten (15) mittels einer Bildgebungsvorrichtung (1), umfassend:
- Erfassen (S1) einer Information (I1) über die Körperregion (31),
- Bereitstellen (S2) einer Eingabeoption (E1) zum Erfassen einer Information (I2) über einen Subbereich (33) der Körperregion (15) in Abhängigkeit der Information (I1) über die Körperregion (31), wobei die Eingabeoption (E1) eine Mehrzahl von Subbereichen (33a,33b) der Körperregion (15) umfasst,
- Erfassen (S3) des diagnostisch relevanten Subbereichs (32) in Abhängigkeit der Eingabeoption (E1) zum Erfassen der Information (I2) über den Subbereich (33a) der Körperregion (15),
- Bestimmen (S4) des Bildgebungsprotokolls zur Erfassung von Bilddaten des diagnostisch relevanten Subbereichs (32) der Körperregion (31) des Patienten (15) in Abhängigkeit des erfassten diagnostisch relevanten Subbereichs (32),
- Bereitstellen (S5) des Bildgebungsprotokolls zur Erfassung von Bilddaten des diagnostisch relevanten Subbereichs (32) der Körperregion (31) mittels der Bildgebungsvorrichtung (1).

2. Verfahren nach Anspruch 1, wobei das Erfassen (S1) der Information (I1) über die Körperregion (31) ein Erfassen mindestens einer der folgenden Informationen umfasst: Patientendaten, Bilddaten aus vorangegangenen Untersuchungen, insbesondere der Körperregion des Patienten, eine Untersuchungsanweisung.

3. Verfahren nach einem der vorhergehenden Ansprüche, wobei das Ermitteln (S4) des Bildgebungsprotokolls zur Erfassung von Bilddaten des diagnostisch relevanten Subbereichs (32) der Körperregion (31) des Patienten (15) in Abhängigkeit des erfassten diagnostisch relevanten Subbereichs (32) ein Auswählen eines Bildgebungsprotokolls aus einer Mehrzahl von in einer Datenbank gespeicherten Bildgebungsprotokollen umfasst.

4. Verfahren nach einem der vorhergehenden Ansprüche, wobei das Ermitteln (S4) des Bildgebungsprotokolls zur Erfassung von Bilddaten des diagnostisch relevanten Subbereichs (32) der Körperregion (31) des Patienten (15) in Abhängigkeit des erfassten diagnostisch relevanten Subbereichs (32) ein Bestimmen eines Bildgebungsparameters des Bildgebungsprotokolls umfasst.

5. Verfahren nach einem der vorhergehenden Ansprüche, wobei das Erfassen (S3) des diagnostisch relevanten Subbereichs (32) in Abhängigkeit der Eingabeoption (E1) zum Erfassen der Information über den Subbereich (33) der Körperregion (15)
- ein Erfassen einer Mehrzahl diagnostisch relevanter Subbereiche (32a, 32b) umfasst,
wobei das Ermitteln (S4) des Bildgebungsprotokolls zur Erfassung von Bilddaten des diagnostisch relevanten Subbereichs (32) der Körperregion (31) des Patienten (15) in Abhängigkeit des erfassten diagnostisch relevanten Subbereichs (32) ein Ermitteln eines Bildgebungsprotokolls für jeden der erfassten diagnostisch relevanten Subbereiche (32a,32b) umfasst, und
wobei das Bereitstellen (S5) des Bildgebungsprotokolls für die Erfassung von Bilddaten des diagnostisch relevanten Subbereichs (32) der Körperregion (15) mittels der Bildgebungsvorrichtung (1) ein Bereitstellen eines Bildgebungsprotokolls für jeden der erfassten diagnostisch relevanten Subbereiche (32a,32b) umfasst.

6. Verfahren nach einem der vorhergehenden Ansprüche 1 bis 4, wobei das Erfassen (S3) des diagnostisch relevanten Subbereichs (32) in Abhängigkeit der Eingabeoption (E1) zum Erfassen der Information (I2) über den Subbereich (33) der Körperregion (15) ein Erfassen einer Mehrzahl diagnostisch relevanter Subbereiche (32a,32b) umfasst,
wobei das Verfahren ferner aufweist:
- ein Bestimmen eines gemeinsamen Bildgebungsbereichs (35) der Körperregion (31) anhand der Mehrzahl erfasster diagnostisch relevanter Subbereiche (32a,32b),
wobei das Ermitteln (S4) des Bildgebungsprotokolls zur Erfassung von Bilddaten der diagnostisch relevanten Subbereiche (32a,32b) der Körperregion (31) des Patienten (15) in Abhängigkeit des gemeinsamen Bildgebungsbereichs (35) erfolgt.

7. Verfahren nach einem der vorhergehenden Ansprüche, ferner umfassend:
- Bereitstellen (S6) einer weiteren Eingabeoption (E2) zum Erfassen einer weiteren Information (I3) über den diagnostisch relevanten Subbereich (32),
- Erfassen (S7) einer Eingabe in Abhängigkeit der Eingabeoption (E2) zum Erfassen der weiteren Information (I3) über den diagnostisch relevanten Subbereich (32),
wobei das Ermitteln (S4) des Bildgebungsprotokolls zur Erfassung von Bilddaten des diagnostisch relevanten Subbereichs (32) der Körperregion (31) des Patienten (15) zusätzlich in Abhängigkeit der weiteren Information (I3) erfolgt.

8. Verfahren nach Anspruch 7, wobei die weitere Eingabeoption (E2) zum Erfassen der weiteren Information (I3) über den diagnostisch relevanten Subbereich (32) zumindest eines der folgenden Informationen umfasst:
einen Bildgebungsparameter des Bildgebungsprotokolls, eine Anzahl von Bildgebungsuntersuchungen, eine Reihenfolge von Bildgebungsuntersuchungen, einen Teilbereich des Subbereichs (33) der Körperregion (31) des Patienten (15).

9. Verfahren nach Anspruch 7, wobei das Bereitstellen der weiteren Eingabeoption (E2) zum Erfassen der Information (I3) über den diagnostisch relevanten Subbereich (32) ein Ermitteln eines vorläufigen Bildgebungsbereichs umfasst,
wobei der vorläufige Bildgebungsbereich an den diagnostisch relevanten Subbereich (32) angepasst ist.

10. Verfahren nach einem der vorhergehenden Ansprüche, wobei das Bereitstellen (S2) der Eingabeoption (E1) zum Erfassen der Information (I2) über den Subbereich (33) der Körperregion (15) in Abhängigkeit der Information (I1) über die Körperregion (31) ein Bereitstellen zumindest eines der folgenden Eingabeoptionen umfasst:
eine grafische Repräsentation der Körperregion (31), eine grafische Auswahl des Subbereichs (33) der Körperregion (31), eine textbasierte Auswahl des Subbereichs (33) der Körperregion (31) umfasst.

11. Verfahren nach Anspruch 10, wobei die grafische Repräsentation der Körperregion (31) und/oder die grafische Auswahl des Subbereichs (33) der Körperregion (31) eine zweidimensionale Repräsentation, insbesondere eine Schnittrepräsentation, der Körperregion (31) und/oder der Subbereiche (33) der Körperregion (31) umfasst.

12. Verfahren nach einem der vorhergehenden Ansprüche, wobei die Körperregion (31) des Patienten (15) eine Kieferregion und/oder eine Zahnregion umfasst.

13. Bildgebungsvorrichtung zur Erfassung von Bilddaten eines diagnostisch relevanten Subbereichs (32) einer Körperregion (31) eines Patienten (15), umfassend eine Steuereinheit (22), eine Ausgabeeinheit (23; 24) und eine Benutzerschnittstelle (23; 25),
wobei die Steuereinheit (22) dazu ausgebildet ist, ein Verfahren nach einem der vorhergehenden Ansprüche mittels der Bildgebungsvorrichtung (1) auszuführen,
wobei die Ausgabeeinheit (23; 24) dazu ausgebildet ist, einem Nutzer der Bildgebungsvorrichtung (1) die Eingabeoption (E1) zum Erfassen der Information (I2) über den Subbereich (33) der Körperregion (31) bereitzustellen, und
wobei die Benutzerschnittstelle (23; 25) dazu ausgebildet ist, das Bildgebungsprotokoll in Abhängigkeit des diagnostisch relevanten Subbereichs (32) bereitzustellen.

14. Bildgebungsvorrichtung nach Anspruch 13, wobei die Bildgebungsvorrichtung (1) als eine Magnetresonanzvorrichtung (10) ausgestaltet ist.

15. Computerprogrammprodukt, welches direkt in eine Speichereinheit (29) einer Recheneinheit (28) einer Bildgebungsvorrichtung (1) nach einem der Ansprüche 13 oder 14 ladbar ist, mit Programmcode-Mitteln, um ein Computer-implementiertes Verfahren nach einem der Ansprüche 1 bis 12 auszuführen, wenn das Computerprogrammprodukt in der Recheneinheit (28) der Bildgebungsvorrichtung (1) ausgeführt wird.
